# EUROPÄISCHE PATENTSCHRIFT

(11) **EP 2 063 938 B1**
(45) Veröffentlichungstag und Bekanntmachung des Hinweises auf die Patenterteilung: **05.04.2017**
(21) Anmeldenummer: 07785109.5
(22) Anmeldetag: 22.08.2007
(51) Int. Cl.: A61M 5/32

(54) **NADELSCHUTZVORRICHTUNG MIT DISTALEM UND PROXIMALEM NADELSCHUTZ**
NEEDLE PROTECTION DEVICE COMPRISING A DISTAL PROTECTION ELEMENT AND A PROXIMAL PROTECTION ELEMENT
DISPOSITIF DE PROTECTION D'AIGUILLE COMPRENANT UNE PROTECTION DISTALE ET UNE PROTECTION PROXIMALE

(30) Priorität: 06.09.2006 DE 102006042233
(43) Veröffentlichungstag der Anmeldung: 03.06.2009
(73) Patentinhaber: TecPharma Licensing AG, 3400 Burgdorf (CH)
(72) Erfinder: GRATWOHL, Christian, 5000 Aarau (CH); WYMANN, Martin, 3097 Liebefeld (CH); LANZ, Marc, 3268 Lobsigen (CH); WIDMER, Urs, 3013 Bern (CH)
(86) Internationale Anmeldenummer: PCT/CH2007/000414
(87) Internationale Veröffentlichungsnummer: WO 2008/028305

(56) Entgegenhaltungen:
- WO-A-01/91837
- WO-A-01/93924
- WO-A-03/045480
- WO-A-2004/030539

## Beschreibung

Die Erfindung betrifft eine Nadelschutzvorrichtung, die an einem Injektionsgerät lösbar befestigt oder befestigbar ist. Die Nadelschutzvorrichtung findet bevorzugt in der Verabreichung von Medikamenten Verwendung, beispielsweise der Verabreichung von Insulin, und kann insbesondere in der Selbstverabreichung verwendet werden, d.h. von Patienten, die sich das betreffende Medikament selbst verabreichen. Bei dem Injektionsgerät handelt es sich vorzugsweise um eines für den wiederholten Gebrauch, und besonders bevorzugt handelt es sich um ein Injektionsgerät, das die Einstellung der Dosis des zu verabreichenden Produkts zulässt. Das Injektionsgerät kann insbesondere ein Injektionspen sein, wie er beispielsweise in der Diabetestherapie und mittlerweile auch anderen Therapien verwendet wird.

Um die Gefahr von Infektionen zu vermeiden, wurden Nadelschutzvorrichtungen entwickelt, die nur den einmaligen Gebrauch einer Injektionsnadel zulassen. Eine derartige Nadelschutzvorrichtung ist beispielsweise aus der WO2001/91837A1 bekannt. Die Injektionsnadel durchragt den Nadelhalter und wird von dem Nadelhalter festgehalten. Sie weist einen in die distale Richtung über den Nadelhalter hinausragenden Injektionsabschnitt und einen in die proximale Richtung über den Nadelhalter hinausragenden Verbindungsabschnitt auf.

Aus dem Stand der Technik sind ferner die folgenden Nadelschutzvorrichtung bekannt, nämlich aus der WO2004/030539A1 ist eine Aufnahmevorrichtung, insbesondere ein Blutprobenentnahmeröhrchen, mit einem verstellbaren Abdeckelement bekannt, wobei eine Arretiereinrichtung die Bewegung des Abdeckelements blockieren kann. Aus der WO2001/93924A1 ist eine medizinische Vorrichtung mit einer Nadeleinrichtung bekannt, wobei der Benutzer nach dem Benutzen der medizinischen Vorrichtung einen Betätigungsknopf bedienen kann, um die Nadeleinrichtung mittels einer Feder in die medizinische Vorrichtung zu bringen. Aus der WO03/045480A1 ist eine Sicherheitsnadel mit einer Injektionsnadel bekannt, wobei ein distaler Nadelschutz den distalen Teil der Injektionsnadel überdecken kann.

Aus dem Stand der Technik sind ferner die folgenden Nadelschutzvorrichtung bekannt, nämlich aus der WO2004/030539A1 ist eine Aufnahmevorrichtung, insbesondere ein Blutprobenentnahmeröhrchen, mit einem verstellbaren Abdeckelement bekannt, wobei eine Arretiereinrichtung die Bewegung des Abdeckelements blockieren kann. Aus der WO 01/93924A1 ist ein medizinische Vorrichtung mit einer Nadeleinrichtung bekannt, wobei der Benutzer nach dem Benutzen der medizinischen Vorrichtung einen Betätigungsknopf bedienen kann, um die Nadeleinrichtung mittels einer Feder in die medizinische Vorrichtung zu bringen. Aus der WO03/045480A1 ist eine Sicherheitsnadel mit einer Injektionsnadel bekannt, wobei ein distaler Nadelschutz den distalen Teil der Injektionsnadel überdecken kann.

Es ist eine Aufgabe der Erfindung, die Sicherheit vor Stichverletzungen bei Nadelschutzvorrichtungen zu vergrössern.

Die Erfindung betrifft eine Nadelschutzvorrichtung, die an einem Injektionsgerät lösbar befestigt ist oder erst noch befestigt wird. Die Nadelschutzvorrichtung umfasst eine Injektionsnadel und einen die Injektionsnadel haltenden Nadelhalter, über den die

Injektionsnadel mit einem Nadelinjektionsabschnitt in die distale Richtung und mit einem Nadelverbindungsabschnitt in die proximale Richtung vorragt. Die Nadelschutzvorrichtung umfasst ferner einen mit dem Nadelhalter beweglich verbundenen distalen Nadelschutz für den Nadelinjektionsabschnitt. Vorzugsweise durchragt die Injektionsnadel den Nadelhalter und wird vom Nadelhalter festgehalten. Alternativ können der Nadelinjektionsabschnitt und der Nadelverbindungsabschnitt auch separate Nadeln sein, die vom Nadelhalter gehalten und fluidisch miteinander verbunden werden. Grundsätzlich kann der Nadelhalter die beiden Nadelabschnitte auch in einem Stück bilden. Der Nadelinjektionsabschnitt wird bei der Verabreichung des jeweiligen Produkts durch die Haut und vorzugsweise subkutan in das darunter liegende Gewebe gestochen. Der Nadelverbindungsabschnitt durchsticht bei dem Befestigen eine Membran, die ein mit dem zu verabreichenden Produkt gefülltes Reservoir an einem distalen Auslass dicht verschließt. Der distale Nadelschutz ist relativ zu dem Nadelhalter aus einer Freigabeposition in die distale Richtung bis in eine Schutzposition bewegbar, vorzugsweise mittels Federkraft. Wenn der Nadelschutz die Freigabeposition einnimmt, steht die Injektionsnadel mit ihrem Nadelinjektionsabschnitt über den Nadelschutz in die distale Richtung vor. In der Schutzposition hingegen überlappt der Nadelschutz den Nadelinjektionsabschnitt bis einschließlich zu einem distalen Ende der Injektionsnadel. Bevorzugt wird es, wenn der Nadelschutz in einem Ausgangszustand der Vorrichtung, vor dem ersten Gebrauch, eine distale Ausgangsposition einnimmt, aus der er in die Freigabeposition bewegt werden kann.

In bevorzugten Ausführungen ist der distale Nadelschutz in der Schutzposition blockiert, so dass er nicht wieder in die Freigabeposition bewegt werden kann. Die Blockierung wird vorzugsweise automatisch hergestellt, wenn der Nadelschutz bei der Bewegung in die distale Richtung die Schutzposition erreicht hat. Über derart selbst blockierende Nadelschutzvorrichtungen hinaus betrifft die Erfindung grundsätzlich jedoch auch Nadelschutzvorrichtungen mit beweglichem distalen Nadelschutz, der nach einer Injektion nicht blockiert. In solchen Ausführungen dient der Nadelschutz in erster Linie nur als Sichtschutz, um einem Verwender, der sich das Produkt selbst verabreicht, die Angst vor der Injektionsnadel zu nehmen.

Nach der Erfindung ist die Nadelschutzvorrichtung auch noch mit einem proximalen Nadelschutz ausgestattet, der beweglich mit dem Nadelhalter verbunden ist. Der proximale Nadelschutz kann aus einer Freigabeposition in die proximale Richtung bis in eine Schutzposition bewegt werden. In der Freigabeposition ragt der Nadelverbindungsabschnitt in die proximale Richtung über den proximalen Nadelschutz vor. In der Schutzposition überlappt der proximale Nadelschutz den Nadelverbindungsabschnitt bis einschließlich zu dem proximalen Ende der Injektionsnadel. Die Nadelschutzvorrichtung umfasst ferner eine Blockiereinrichtung für den proximalen Nadelschutz. Sobald der proximale Nadelschutz bei einer Bewegung in die proximale Richtung die Schutzposition erreicht hat, wird er automatisch von der Blockiereinrichtung blockiert, so dass er sich nicht wieder zurück in die Freigabeposition bewegen kann. Während Nadelschutzvorrichtungen mit distalem Nadelschutz aus dem Stand der Technik, beispielsweise der bereits genannten WO 01/91837 A1, bekannt sind, hat erstmals die Erfindung erkannt, dass auch der Nadelverbindungsabschnitt nach der Benutzung der jeweiligen Nadelschutzvorrichtung Stichverletzungen verursachen kann und eliminiert diese Gefahr mittels des weiteren Nadelschutzes, den die erfindungsgemäße Nadelschutzvorrichtung nach Gebrauch, d.h. bei oder nach dem Entfernen von dem Injektionsgerät, automatisch in seiner Schutzposition blockiert.

Die Nadelschutzvorrichtung umfasst eine Befestigungseinrichtung für die lösbare Verbindung mit dem Injektionsgerät. Die Befestigungseinrichtung wirkt vorzugsweise mit am Injektionspen außen vorgesehenen Befestigungsmitteln zusammen. Sie kann beispielsweise eine Schraub- oder Bajonetthülse oder eine Steck-, Rast- oder Klipphülse sein. In derartigen Ausführungen umgibt die Befestigungseinrichtung zwar den Nadelverbindungsabschnitt, wie dies grundsätzlich von bekannten Nadelschutzvorrichtungen bekannt ist. Im allgemeinen sind die bekannten Befestigungseinrichtungen im Durchmesser jedoch so groß, dass der Verwender problemlos mit dem Finger die Nadelspitze des Nadelverbindungsabschnitts erreichen und sich verletzen kann. Der erfindungsgemäße distale Nadelschutz ist vorteilhafterweise jedoch näher als derartige Befestigungseinrichtungen bei dem Nadelverbindungsabschnitt angeordnet, wobei der quer zu dem Nadelverbindungsabschnitt gemessene Abstand so klein ist, dass der Verwender mit der proximalen Nadelspitze nicht in Kontakt kommt, wenn er das proximale Ende des proximalen Nadelschutzes berührt. Der Hersteller hat wegen des erfindungsgemäßen Nadelschutzes auch größere Freiheit in der Gestaltung der Befestigungseinrichtung, da eine zusätzliche Schutzfunktion wie bevorzugt hülsenförmige Befestigungseinrichtungen sie bieten, zwar nach wie vor vorteilhaft ist, der Schutz vor Stichverletzungen jedoch auch einzig und allein durch den erfindungsgemäßen proximalen Nadelschutz erfolgen kann.

In bevorzugten Ausführungen umfasst die Nadelschutzvorrichtung ein Federglied, das den proximalen Nadelschutz in die proximale Richtung mit einer Federkraft beaufschlagt. Das Federglied stützt sich vorzugsweise unmittelbar an dem proximalen Nadelschutz ab, könnte grundsätzlich jedoch auch über ein oder mehrere Zwischenglieder erst auf den Nadelschutz wirken. In derartigen Ausführungen wird der Nadelschutz bei der Entfernung der Nadelschutzvorrichtung von dem Injektionsgerät durch Federkraft in die Schutzposition bewegt. In Weiterentwicklungen wirkt das gleiche Federglied auch noch in die distale Richtung auf den distalen Nadelschutz, vorzugsweise unmittelbar oder gegebenenfalls über ein oder mehrere Zwischenglieder. In besonders bevorzugten Ausführungen stützt sich das Federglied an einem Ende an dem distalen Nadelschutz und an den gegenüberliegenden Ende an dem proximalen Nadelschutz ab. Das Federglied kann der Funktion nach insbesondere eine Druckfeder sein. Der Form nach ist es vorzugsweise eine Spiralfeder. In alternativen Ausführungen wird der proximale Nadelschutz nicht durch Federkraft in die Schutzposition bewegt, sondern mittels einer Rückhalteeinrichtung, die einen Rückhalter am proximalen Nadelschutz und einen Rückhalter am Injektionsgerät oder Produktreservoir umfasst, die bei dem Verbinden der Nadelschutzvorrichtung und dem Injektionsgerät automatisch in einen Rückhalteeingriff gelangen, der bei dem Lösen der Nadelschutzvorrichtung bewirkt, dass sich der proximale Nadelschutz aus der Freigabeposition in die Schutzposition bewegt Nimmt der proximale Nadelschutz erst einmal seine Schutzposition ein, löst sich der Rückhalteeingriff automatisch, wenn die Nadelschutzvorrichtung gänzlich von dem Injektionsgerät abgenommen wird.

Die Blockiereinrichtung umfasst wenigstens zwei Blockierglieder, nämlich ein erstes Blockierglied, das an dem Nadelhalter geformt oder im Falle einer separaten Formung mit dem Nadelhalter verbunden ist, und ein zweites Blockierglied, das an dem proximalen Nadelschutz geformt oder bei separater Formung mit dem Nadelschutz verbunden ist. Vorzugsweise ist wenigstens eines der Blockierglieder gegen eine rückstellende Federkraft quer zu der Längsrichtung der Injektionsnadel beweglich. Das betreffende Blockierglied selbst kann unnachgiebig, d.h. in sich steif sein und wird in derartigen Ausführungen von einem separaten Federglied mit der rückstellenden Federkraft beaufschlagt. Vorzugsweise ist das betreffende Blockierglied jedoch formelastisch, besonders bevorzugt bildet es einen elastischen Biegebalken. Die rückstellende Federkraft dient insbesondere dazu, das Blockierglied in den Blockiereingriff mit dem anderen Blockierglied zu bewegen. Der Blockiereingriff wird vorzugsweise durch eine elastische Schnappbewegung bewirkt. In alternativen Ausführungen können aber auch nur in sich steife, d.h. nicht nachgiebige Blockierglieder die Blockiereinrichtung bilden. Allerdings muss dafür gesorgt sein, dass die Blockierglieder in den Blockiereingriff bewegt werden, wenn sich der proximale Nadelschutz in die Schutzposition bewegt. In solchen Ausführungen kann die Blockiereinrichtung eine oder mehrere Kulissenführungen umfassen, mittels der oder denen die Blockierglieder relativ zueinander zwangsgeführt in den Blockiereingriff bewegt werden.

Das an dem proximalen Nadelschutz geformte oder mit dem Nadelschutz verbundene zweite Blockierglied kann von dem Nadelschutz aus nach außen geführt und die Nadelschutzvorrichtung sozusagen umgreifend mit dem ersten Blockierglied zusammenwirken, bevorzugter durchragt der proximale Nadelschutz jedoch den Nadelhalter zumindest in der Schutzposition in die distale oder in die proximale Richtung.

In einer ersten Ausführung nimmt der proximale Nadelschutz vor der Benutzung der Nadelschutzvorrichtung eine proximale Ausgangsposition ein, aus welcher er bei dem Verbinden der Nadelschutzvorrichtung mit dem Injektionsgerät in die Freigabeposition bewegt wird. In einer zweiten Ausführung nimmt der proximale Nadelschutz bei der Auslieferung der Nadelschutzvorrichtung bereits die Freigabeposition ein. Die Nadelschutzvorrichtung verfügt in beiden Ausführungen vorzugsweise über ein Deblockierglied. In der ersten Ausführung wirkt das Deblockierglied mit dem wenigstens einen quer zur Längsrichtung der Injektionsnadel beweglichen Blockierglied so zusammen, dass der Blockiereingriff nicht bereits bei der Bewegung aus der proximalen Ausgangsposition in die Freigabeposition hergestellt werden kann, indem das Deblockierglied mit dem querbeweglichen Blockierglied in einem Eingriff ist, was auch den Fall einschließen kann, dass es mit dem ersten und dem zweiten Blockierglied in einem den Blockiereingriff verhindernden Eingriff ist. Im Verlaufe der Injektion, vorzugsweise beim Einstechen des Nadelinjektionsabschnitts oder bei oder nach dem Herausziehen des Nadelinjektionsabschnitts aus dem Gewebe löst sich der Eingriff von Deblockierglied und Blockierglied vorteilhafterweise automatisch, so dass die Blockierglieder in den Blockiereingriff gelangen können, wenn die Nadelschutzvorrichtung von dem Injektionsgerät abgenommen wird. Das Deblockierglied wird in der zweiten Ausführung durch das Einstechen der Injektionsnadel aus einer Deblockierposition, in der es eine Bewegung des proximalen Nadelschutzes in die proximale Richtung verhindert, in eine Neutralposition bewegt, in der es eine solche Bewegung und damit eine Bewegung in die Schutzposition zulässt. Das Deblockierglied der zweiten Ausführung kann insbesondere drehbar, vorzugsweise um die Injektionsnadel drehbar, mit dem Nadelhalter verbunden sein.

Die Deblockierglieder beider Ausführungen sind vorzugsweise mit dem distalen Nadelschutz gekoppelt oder werden bei dem Einstechen oder dem Herausziehen der Injektionsnadel aus dem Gewebe automatisch mit dem distalen Nadelschutz gekoppelt. Der distale Nadelschutz bewirkt in derartigen Weiterbildungen über die Kopplung die Bewegung des Deblockierglieds aus der Deblockierposition in die Neutralposition. Die Kopplung kann insbesondere ein Mitnahmeeingriff sein, mittles dem der distale Nadelschutz bei seiner Bewegung in die distale Richtung, d.h. bei dem Herausziehen aus dem Gewebe, das Deblockierglied bis in die Neutralposition mitnimmt. Alternativ kann eine Kulissenführung die Kopplung bilden, wobei die Kulissenführung die Einstechbewegung oder die Ausziehbewegung des distalen Nadelschutzes in die Bewegung des Deblockierglieds aus der Deblockierposition in die Neutralposition umwandelt. So kann mittels der Kulissenführung beispielsweise eine lineare Einstechbewegung oder Ausziehbewegung des distalen Nadelschutzes in eine Drehbewegung des Deblockierglieds umgewandelt werden.

Vorteilhafte Merkmale werden auch in den Unteransprüchen und deren Kombinationen offenbart.

Nachfolgend werden Ausführungsbeispiele der Erfindung anhand von Figuren erläutert. An den Ausführungsbeispielen offenbar werdende Merkmale bilden je einzeln und in jeder Kombination die Gegenstände der Ansprüche und auch die vorstehend beschriebenen Ausgestaltungen vorteilhaft weiter. Es zeigen:
- Figur 1: eine erfindungsgemäße Nadelschutzvorrichtung eines ersten Ausführungsbeispiels in einer Ansicht auf eine distale Stirnseite,
- Figur 2: die Nadelschutzvorrichtung in einem Längsschnitt A-A,
- Figur 3: die Nadelschutzvorrichtung in einem Längsschnitt B-B,
- Figur 4: die Nadelschutzvorrichtung in einem Längsschnitt C-C,
- Figur 5: Komponenten der Nadelschutzvorrichtung aufgereiht längs einer Längsachse der Nadelschutzvorrichtung,
- Figur 6: eine Befestigungs- und Führungseinrichtung der Nadelschutzvorrichtung,
- Figur 7: einen distalen Nadelschutz der Nadelschutzvorrichtung,
- Figur 8: ein Deblockierglied der Nadelschutzvorrichtung,
- Figur 9: einen Nadelhalter der Nadelschutzvorrichtung in perspektivischer Darstellung,
- Figur 10 den: Nadelhalter in einem Längsschnitt,
- Figur 11: einen proximalen Nadelschutz der Nadelschutzvorrichtung in einer perspektivischen Darstellung,
- Figur 12: den proximalen Nadelschutz in einem Längsschnitt,
- Figur 13: eine Blockiereinrichtung der Nadelschutzvorrichtung mit dem proximalen Nadelschutz in einer proximalen Ausgangsposition,
- Figur 14: die Blockiereinrichtung mit dem proximalen Nadelschutz in einer Freigabeposition,
- Figur 15: die Blockiereinrichtung mit dem proximalen Nadelschutz in einer Schutzposition,
- Figur 16: die Blockiereinrichtung in vergrößerter Darstellung mit dem proximalem Nadelschutz in zwei unterschiedlichen Positionen,
- Figur 17: Komponenten einer Nadelschutzvorrichtung eines zweiten Ausführungsbeispiels,
- Figur 18: den distalen Nadelschutz des zweiten Ausführungsbeispiels,
- Figur 19: einen Nadelhalter des zweiten Ausführungsbeispiels in perspektivischer Darstellung,
- Figur 20: den Nadelhalter des zweiten Ausführungsbeispiels in einem Längsschnitt,
- Figur 21: ein Deblockierglied des zweiten Ausführungsbeispiels in perspektivischer Darstellung,
- Figur 22: das Deblockierglied des zweiten Ausführungsbeispiels in einer Draufsicht in die distale Richtung,
- Figur 23: einen proximalen Nadelschutz des zweiten Ausführungsbeispiels,
- Figur 24: eine Blockiereinrichtung des zweiten Ausführungsbeispiels in einem Ausgangszustand vor einer Injektion,
- Figur 25: die Blockiereinrichtung des zweiten Ausführungsbeispiels in einem Endzustand nach einer Injektion,
- Figur 26: die Nadelschutzvorrichtung des zweiten Ausführungsbeispiels in dem Ausgangszustand,
- Figur 27: die Nadelschutzvorrichtung des zweiten Ausführungsbeispiels in einem Zustand während einer Injektion und
- Figur 28: die Nadelschutzvorrichtung des zweiten Ausführungsbeispiels in dem Endzustand.

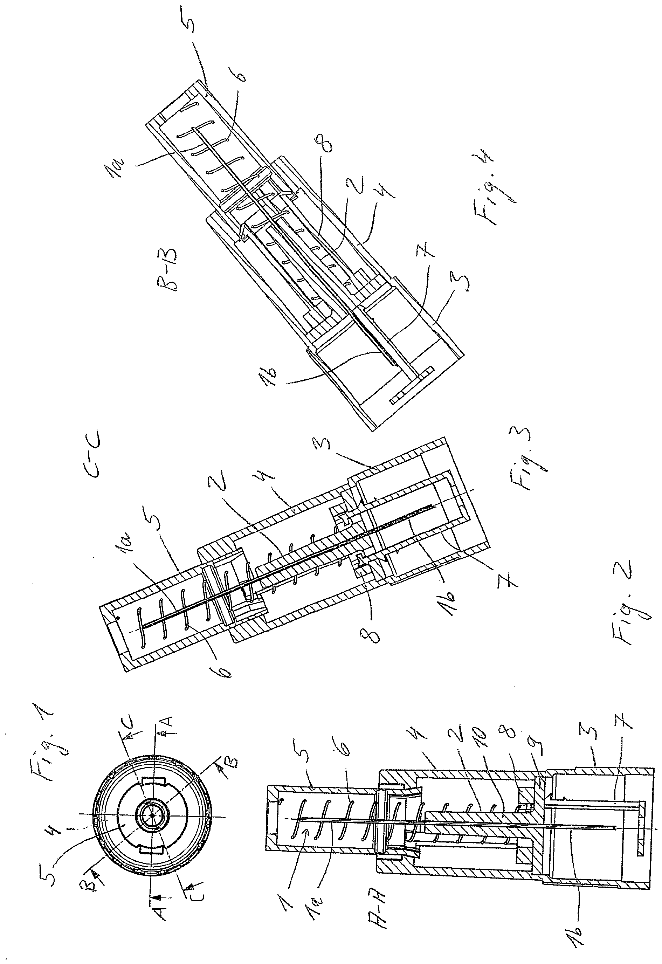

Figur 1 zeigt eine Nadelschutzvorrichtung eines ersten Ausführungsbeispiels in einer Draufsicht auf ein distales Stirnende. Eingetragen sind drei Längsschnittebenen A-A, B-B und C-C.

Figur 2 zeigt die Nadelschutzvorrichtung des ersten Ausführungsbeispiels in dem Längsschnitt A-A der Figur 1. Figur 3 zeigt die Nadelschutzvorrichtung in dem Längsschnitt B-B, und Figur 4 zeigt den Längsschnitt C-C.

Die Figuren 1 bis 4 zeigen die Nadelschutzvorrichtung in einem Ausgangszustand, in dem sie sich vor einem ersten Gebrauch befindet. In diesem Zustand gelangt die Nadelschutzvorrichtung in einer nicht dargestellten sterilen Verpackung zum Verwender. Die Nadelschutzvorrichtung umfasst eine Injektionsnadel 1, die als hohle, durchgehend gerade Kanüle gebildet ist, und einen Nadelhalter 2, der die Injektionsnadel 1 in einem mittleren Nadelabschnitt fest hält, so dass die Injektionsnadel 1 zumindest axial, d.h. in Längsrichtung L, und vorzugsweise auch nicht drehbar fixiert ist. Der Nadelhalter 2 weist einen Boden 9 und einen Haltebereich 10 auf, der zentral von dem Boden 9 abragt und die Injektionsnadel 1 hält. Die Injektionsnadel 1 durchragt den Haltebereich 10 des Nadelhalters 2. Sie ragt mit einem Injektionsabschnitt la in die distale Richtung und mit einem Verbindungsabschnitt 1b in die proximale Richtung über den Haltebereich 10 hinaus.

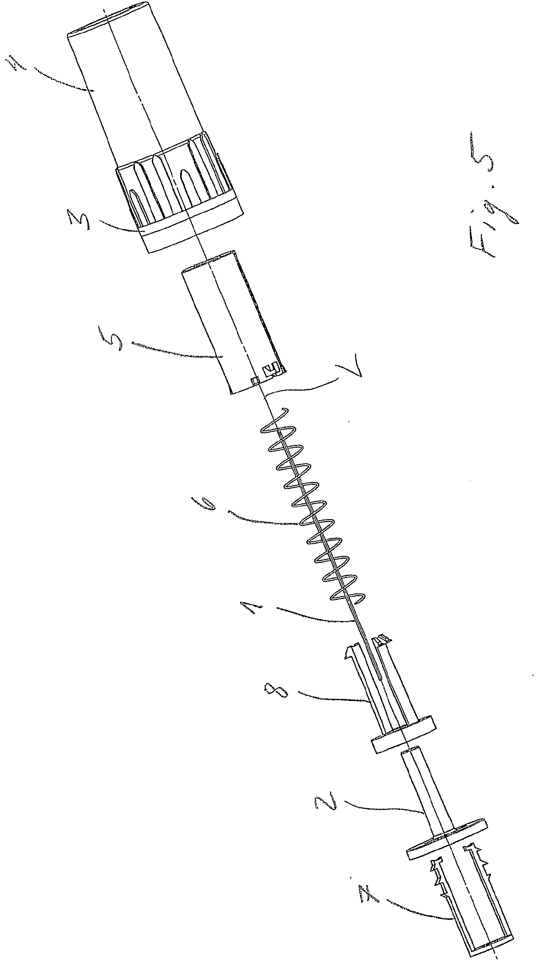

Der Nadelhalter 2 ist in einer hülsenförmigen Befestigungs- und Führungseinrichtung eingesetzt und nicht beweglich befestigt. Der Nadelhalter 2 unterteilt die Führungs- und Befestigungseinrichtung in einen proximalen Befestigungsabschnitt 3 und einen distalen Führungsabschnitt 4. Der Nadelinjektionsabschnitt la ragt mit einer für subkutane Injektionen geeigneten Länge über den Führungsabschnitt 4 in die distale Richtung vor. Der Befestigungsabschnitt 3 bildet eine Befestigungseinrichtung für eine lösbare Befestigung an einem distalen Ende eines Injektionsgeräts. Die Befestigungseinrichtung kann vorteilhafterweise ein oder mehrere Rastelemente für eine Rastverbindung mit dem Injektionsgerät aufweisen. Alternativ kann auch ein Schraubgewinde oder ein Bajonett die Befestigungseinrichtung der Nadelschutzvorrichtung bilden. Der Befestigungsabschnitt 3 umgibt den Nadelverbindungsabschnitt 1b und ragt über diesen in die proximale Richtung vor. Der Führungsabschnitt 4 dient der axialen, verdrehgesicherten Führung eines distalen Nadelschutzes 5, der in dem Ausgangszustand relativ zu dem Nadelhalter 2 eine distale Ausgangsposition einnimmt, in der er über den Führungsabschnitt 4 und die distale Spitze der Injektionsnadel 1 hinausragt. Der Nadelschutz 5 ist ein Hülsenkörper, der den Injektionsabschnitt 1a umlaufend umgibt und gleichzeitig auch einen Sichtschutz bildet, so dass der Verwender den Injektionsabschnitt 1a nicht sehen kann. Der Nadelschutz 5 wird von einem Federglied 6 mit einer in die distale Richtung wirkenden Federkraft beaufschlagt. Der Nadelschutz 5 wird mittels eines Deblockierglieds 8 gegen die Kraft des Federglieds 6 relativ zu dem Führungsabschnitt 4 in der distalen Ausgangsposition gehalten.
Aus den Figuren 5 bis 8 ist das Zusammenwirken des Nadelschutzes 5 mit dem Deblockierglied 8 und dem Führungsabschnitt 4 ersichtlich. In Figur 5 sind die Komponenten der Nadelschutzvorrichtung längs deren zentralen Längsachse L in der Reihenfolge hintereinander aufgereiht, in der sie montiert werden. Die Injektionsnadel 1 ist losgelöst von dem Nadelhalter 2 dargestellt, ist aber zum Zeitpunkt der Montage bereits fest mit dem Nadelhalter 2 verbunden.

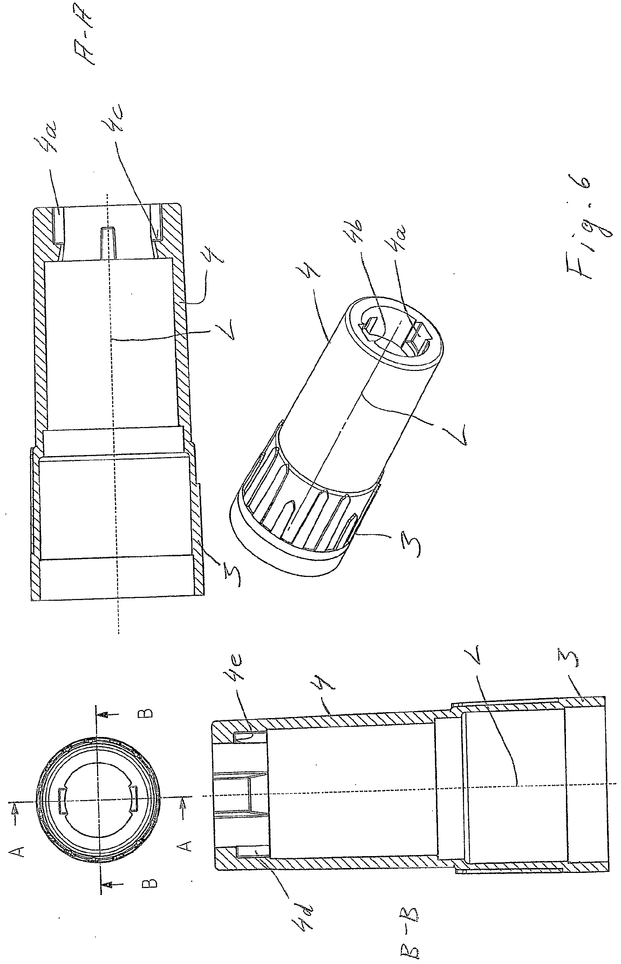

Figur 6 zeigt die Befestigungs- und Führungseinrichtung 3, 4 in einer Ansicht, zwei Längsschnitten A-A und B-B und einer perspektivischen Sicht. Die Befestigungs- und Führungseinrichtung 3, 4 weist in ihrem Führungsabschnitt 4 am distalen Ende in einer um die Längsachse L umlaufenden Mantelinnenfläche zwei Ausnehmungen 4a auf. Die Ausnehmungen 4a gehen je an ihrem proximalen Ende über eine steile Schulter in die Mantelinnenfläche über. Die beiden Schultern bilden je einen in die distale Richtung weisenden Translationsanschlag 4c. Seitlich von den Ausnehmungen 4a sind jeweils axial erstreckte Führungen 4b geformt für die axiale Geradführung des Nadelschutzes 5. An dem distalen Ende des Führungsabschnitts 4 sind in der gleichen Mantelinnenfläche zwei weitere Ausnehmungen 4d geformt, die über den Umfang der Mantelinnenfläche zu den Ausnehmungen 4a jeweils um 90° versetzt sind. Die Ausnehmungen 4d gehen je an ihrem distalen Ende über eine steile Schulter in die Mantelinnenfläche über. Die beiden Schultern bilden je einen in die proximale Richtung weisenden Translationsanschlag 4e.

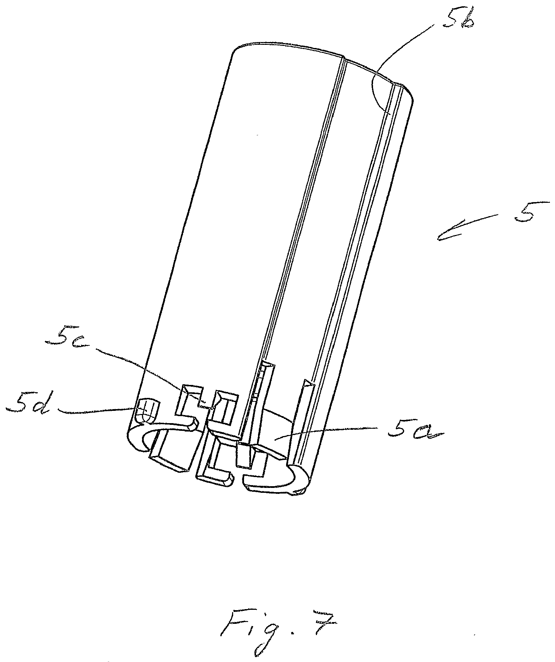

Figur 7 zeigt den Nadelschutz 5. Der Nadelschutz 5 weist axiale Führungen 5b auf, die mit den Führungen 4b des Führungsabschnitts 4 zusammenwirken und mit diesen die verdrehgesicherte Linearführung des Nadelschutzes 5 bilden. Am proximalen Ende des Nadelschutzes 5 sind in dessen Mantel in Umfangsrichtung um 180° zueinander versetzt zwei Durchbrüche gebildet. In die Durchbrüche ragt je in die proximale Richtung ein Vorsprung 5c, der den Mantel des Nadelschutzes 5 in den jeweiligen Durchbruch hinein axial verlängert. Die Innenseiten der Vorsprünge 5b sind in die proximale Richtung zu freien Enden der Vorsprünge 5b hin nach außen geneigt, im Ausführungsbeispiel je mit einer konstanten Neigung. Sie bilden im Zusammenwirken mit dem Deblockierglied 8 je eine Rampe. Der Nadelschutz 5 weist an seinem proximalen Ende ferner in Umfangsrichtung um 180° zueinander versetzt zwei Blockierglieder 5a auf, die im Ausführungsbeispiel als Federzungen gebildet sind. Die Blockierglieder 5a sind in die proximale Richtung nach außen geneigt. Schließlich sind an dem äußeren Umfang des Nadelschutzes 5 ebenfalls an dessen proximalen Ende in Umfangsrichtung um 180° zueinander versetzt zwei Eingriffsglieder 5d als nach außen abragende Nocken geformt.

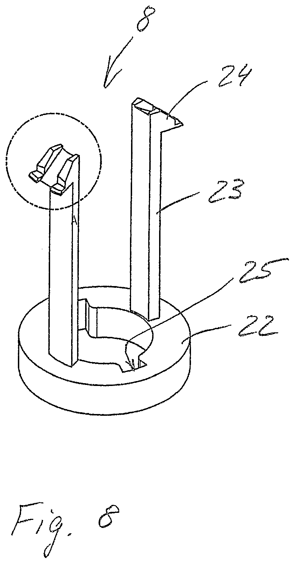

Figur 8 zeigt das Deblockierglied 8. Das Deblockierglied 8 weist an einem proximalen Ende eine ringförmige Basis 22 auf. Von der Basis 22 ragen in die distale Richtung zwei Finger 23 auf, an deren distalen Enden jeweils ein Vorsprung 24 nach außen abragt. Im Ausgangszustand der Nadelschutzvorrichtung ragen die Vorsprünge 24 in die Durchbrüche des Nadelschutzes 5, wie aus Figur 4 ersichtlich, und halten den Nadelschutz 5 gegen die Federkraft des Federglieds 6 in seiner distalen Ausgangsposition, indem das Federglied 6 die äußeren Enden der Vorsprünge 24 auf Anschlag gegen Anschlagflächen des Führungsabschnitts 4 drückt. Die Vorsprünge 24 sind wie die Vorsprünge 5c des Nadelschutzes 5 rampenförmig geneigt und in ihrer Neigung an die Vorsprünge 5c angepasst.

Wie die Figuren 2 bis 4 erkennen lassen, verfügt die Nadelschutzvorrichtung nicht nur über den distalen Nadelschutz 5, sondern auch noch über einen proximalen Nadelschutz 7 für den Verbindungsabschnitt 1b der Injektionsnadel 1. Das Deblockierglied 8 wirkt sowohl mit dem Nadelschutz 5 als auch mit dem Nadelschutz 7 zusammen. Im Zusammenwirken mit dem Nadelschutz 5 erfüllt es die beschriebene Blockierfunktion. Im Zusammenwirken mit dem Nadelschutz 7 erfüllt es eine Deblockierfunktion, indem es in seiner in den Figuren 2 bis 4 gezeigten Ausgangsposition verhindert, dass eine Bewegung des proximalen Nadelschutzes 7 in die distale Richtung blockiert wird. Für das Zusammenwirken mit dem Nadelschutz 7 sind in der Mantelinnenfläche der Basis 22 des Deblockierglieds 8 in Umfangsrichtung um 180° zueinander versetzt zwei axial erstreckte Ausnehmungen geformt, in die der proximale Nadelschutz 7 in seiner proximalen Ausgangsposition eingreift und die für den Nadelschutz 7 je eine axial erstreckte Führungsbahn 25 bilden.

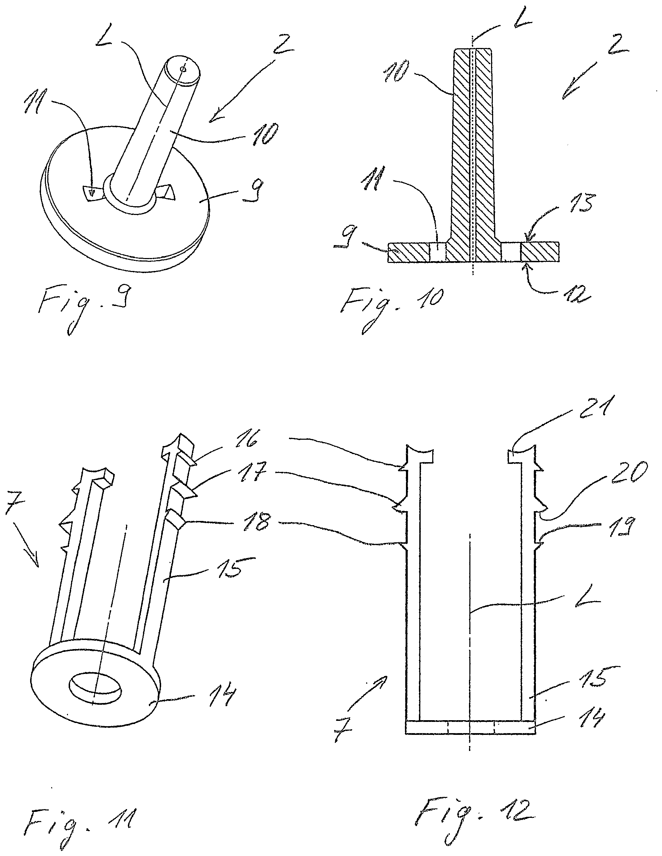

Die Figuren 9 und 10 zeigen den Nadelhalter 2, und die Figuren 11 und 12 zeigen den proximalen Nadelschutz 7. Der Nadelschutz 7 weist an seinem proximalen Ende eine ringförmige Basis 14 und von der Basis 14 in die distale Richtung abragende Blockierglieder 15 auf. Die Blockierglieder 15 sind finger- oder stabförmig. Im Ausführungsbeispiel handelt es sich um zwei Blockierglieder 15, die von einer distalen Stirnseite der Basis 14 abragen und in Umfangsrichtung um die Längsachse L um 180° zueinander versetzt sind, so dass sie in der proximalen Ausgangsposition des Nadelschutzes 7 den Nadelverbindungsabschnitt 1b zwischen sich nehmen. Die Basis 14 weist einen zentralen Durchgang für den Nadelverbindungsabschnitt 1b auf.

In einem distalen Abschnitt des Nadelschutzes 7 sind an den Blockiergliedern 15 je mehrere nach außen abragende Vorsprünge geformt, im Ausführungsbeispiel drei Vorsprünge 16, 17 und 18. Ferner weisen die Blockierglieder 15 je eine Abstützung 21 für das Federglied 6 auf. Die Abstützungen 21 werden von Vorsprüngen gebildet, die an den distalen Enden der Blockierglieder 15 nach innen aufeinander zu ragen. Das Federglied 6 stützt sich über die Abstützungen 21 in die proximale Richtung an dem Nadelschutz 7 ab, ist also zwischen dem Nadelschutz 5 und dem Nadelschutz 7 gespannt.

Im montierten Zustand befindet sich die Basis 14 des Nadelschutzes 7 proximal von dem Boden 9 des Nadelhalters 2, und die stab- oder fingerförmigen Blockierglieder 15 ragen in die distale Richtung durch zwei Durchgänge 11, die in dem Boden 9 des Nadelhalters 2 geformt sind. In der proximalen Ausgangsposition nehmen die distalen Vorsprünge 16 die Kraft des Federglieds 6 auf. Die Vorsprünge 16 bilden zu diesem Zweck je einen in die proximale Richtung weisenden Anschlag, der von dem Federglied 6 gegen einen Gegenanschlag 13 (Figur 10) des Nadelhalters 2 gedrückt wird, den im Ausführungsbeispiel der Boden 9 des Nadelhalters 2 bildet. Der Vorsprung 17 bildet einen weiteren in die proximale Richtung weisenden Anschlag 20. Der Vorsprung 18 bildet noch einen Anschlag 19, der allerdings in die distale Richtung weist.

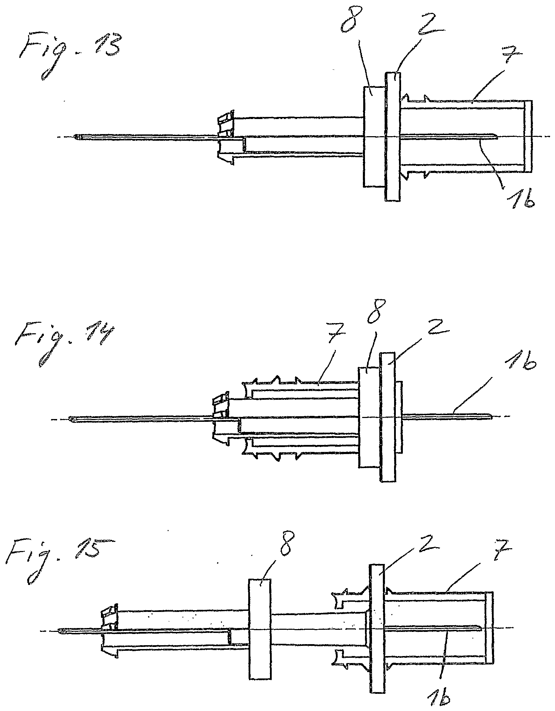

Die Figuren 13, 14 und 15 zeigen die unterschiedlichen Positionen, die der proximale Nadelschutz 7 relativ zu dem Nadelhalter 2 einnimmt, wenn die Nadelschutzvorrichtung mit dem Injektionsgerät verbunden und nach einer Injektion wieder von dem Injektionsgerät gelöst wird. In Figur 13 nimmt der Nadelschutz 7 seine proximale Ausgangsposition ein. Er ist aus der Ausgangsposition gegen die Kraft des Federglieds 6 relativ zu dem Nadelhalter 2 und dem in einer Deblockierposition befindlichen Deblockierglied 8 bis in eine in Figur 14 gezeigte Freigabeposition bewegbar, in welcher der Nadelverbindungsabschnitt 1b in die proximale Richtung über den Nadelschutz 7 vorragt. In Figur 15 nimmt der Nadelschutz 7 eine Schutzposition ein, in der er relativ zu dem Nadelhalter 2 blockiert ist, so dass er aus der Schutzposition nicht wieder in die Freigabeposition bewegt werden kann. Für die Blockierung bilden der Nadelhalter 2 und der Nadelschutz 7 eine Blockiereinrichtung mit im Blockiereingriff befindlichen Blockiergliedern, nämlich den beiden Blockiergliedern 15 des Nadelschutzes 7 und dem Boden 9 als Blockierglied des Nadelhalters 2. Der Nadelschutz 7 ist im Ergebnis ein einziges Mal bis in die Freigabeposition bewegbar, nämlich durch eine auf den Nadelschutz 7 in die distale Richtung ausgeübte äußere Kraft, und bewegt sich bei Fortfall der äußeren Kraft unter der Federkraft des Federglieds 6 automatisch wieder in die proximale Richtung bis in die blockierte Schutzposition. In der Schutzposition ragt er in die distale Richtung über die distale Spitze des Nadelverbindungsabschnitts 1b vor und schützt so den Verwender vor Stichverletzungen. Im Ausführungsbeispiel wird ein besonders sicherer Schutz durch die ringförmige Basis 14 des Nadelschutzes 7 erzielt, deren zentraler Durchgang so eng ist, dass die Verbindungsnadel bei der Befestigung an dem Injektionsgerät den Durchgang passieren kann, andererseits der Verwender aber nicht durch den Durchgang hindurch die distale Nadelspitze erreichen kann.

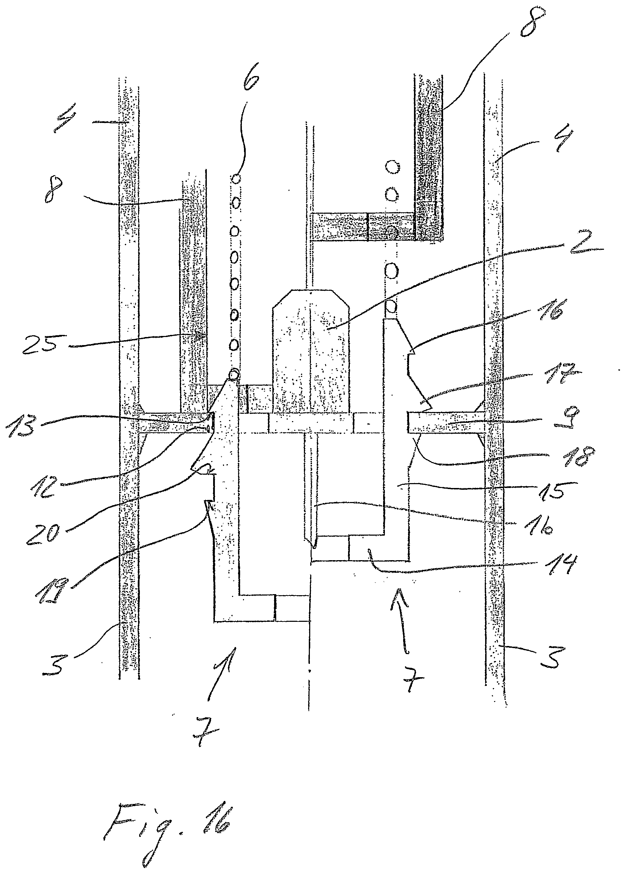

Figur 16 zeigt die bei den Bewegungen des Nadelschutzes 7 zusammenwirkenden Komponenten in zwei Zuständen. In der linken Hälfte der Figur 16 nimmt der Nadelschutz 7 die distale Ausgangsposition und in der rechten Hälfte die blockierte Schutzposition ein. Die linke Hälfte entspricht dem in den Figuren 2 bis 4 und der Figur 13 dargestellten Zustand, und die rechte Hälfte der Figur 16 entspricht dem in Figur 15 dargestellten Zustand.

In der Ausgangsposition halten die Vorsprünge 16 den Nadelschutz 7 am Nadelhalter 2. Die Vorsprünge 17 sind pfeilförmig in die distale Richtung verjüngt, während die Vorsprünge 18 pfeilförmig in die proximale Richtung verjüngt sind. Die Anschläge 19 und 20 der Vorsprünge 17 und 18 sind einander axial zugewandt. Wird die Nadelschutzvorrichtung mittels des Befestigungsabschnitts 3 an dem Injektionsgerät befestigt, beispielsweise auf- oder eingeschraubt oder verklippt, kontaktiert der Nadelschutz 7 das distale Ende des Injektionsgeräts und wird im Verlaufe der Befestigung gegen die Kraft des Federglieds 6 in die distale Richtung gedrückt. Bei dieser Bewegung gleiten die Vorsprünge 17 mit ihren pfeilförmigen distalen Seiten durch die Durchgänge 11 des Nadelhalters 2, so dass die Blockierglieder 15 elastisch nach innen abgebogen werden. Sobald die Vorsprünge 17 die Durchgänge 11 passiert haben, gelangen sie in Kontakt mit den axialen Führungsbahnen 25 des Deblockierglieds 8. Die Blockierglieder 15 bleiben dabei im abgebogenen Zustand, wofür die Durchgänge 11 vorteilhafterweise die Führungsbahnen 25 einfach axial verlängern. Die Vorsprünge 17 ragen weiter nach außen vor als die nun in den Bereich der Durchgänge 11 gelangenden nachfolgenden Vorsprünge 18. Das quer zu der Längsachse L gemessene Übermaß der Vorsprünge 17 ist so groß, dass die Vorsprünge 20 im Kontakt mit den Führungsbahnen 25 die Blockierglieder 15 so weit von dem äußeren Rand der Durchgänge 11 weg halten, dass die Vorsprünge 18 in die distale Richtung ebenfalls durch die Durchgänge 11 bewegt werden können. Nachdem auch die Vorsprünge 18 die Durchgänge 11 passiert haben, bewegt sich der Nadelschutz 7 durch den Kontakt mit dem Injektionsgerät weiter in die distale Richtung bis der Nadelschutz 7 nach Herstellung der Verbindung mit dem Injektionsgerät die in Figur 14 dargestellte Freigabeposition einnimmt. Gleichzeitig durchsticht der Nadelverbindungsabschnitt 1b bei dem Befestigen am Injektionsgerät eine Dichtmembran an einem distalen Ende eines Medikamentenreservoirs und stellt so die Fluidverbindung zwischen dem Medikamentenreservoir und der proximalen Spitze des Nadelinjektionsabschnitts 1a her.

Wird die Nadelschutzvorrichtung von dem Injektionsgerät gelöst und dadurch der proximale Nadelschutz 7 entlastet, drückt das Federglied 6 den Nadelschutz 7 in die proximale Richtung. Die proximalen Vorsprünge 18 gelangen zuerst in Kontakt mit dem Boden 9, der das Blockierglied des Nadelhalters 2 bildet, so dass die Blockierglieder 15 wieder elastisch nach innen abgebogen werden und die Durchgänge 11 in die proximale Richtung passieren können. Damit die am weitesten nach radial außen abragenden Vorsprünge 18 bei der Bewegung des Nadelschutzes 7 in die Schutzsposition das Deblockierglied 8 passieren können, kann die Führungsbahn 25 des Deblockierglieds 8 beispielsweise in die axiale Richtung ausreichend lang sein, wie in Figur 16 dargestellt. Alternativ können bei kurzen Führungsbahnen 25, wie bei dem Deblockierglied der Figuren 8-15, die Vorsprünge 18 an ihren äußeren Enden gerundet oder wie im Ausführungsbeispiel der Figur 16 zusätzlich und ferner auch in den Figuren 12 und 14 erkennbar abgeschrägt sein. In dem in der rechten Hälfte der Figur 16 dargestellten Endzustand nach Gebrauch verhindern die Vorsprünge 18 mit ihrem jeweiligen Anschlag 19 im Zusammenwirken mit dem Boden bzw. Blockierglied 9, dass der Nadelschutz 7 relativ zu dem Nadelhalter 2 wieder in die distale Richtung bewegt werden kann.

Der Gebrauch der Nadelschutzvorrichtung ist wie folgt:
Der Verwender befestigt die im Ausgangszustand der Figuren 2 bis 4 befindliche Nadelschutzvorrichtung an einem Injektionsgerät, indem er den Befestigungsabschnitt 3 mit dem distalen Ende des Injektionsgeräts verbindet. Bei dem Verbinden drückt das Injektionsgerät in die distale Richtung gegen den proximalen Nadelschutz 7, so dass dieser sich in die in Figur 14 dargestellte Freigabeposition bewegt. Gleichzeitig durchdringt die Injektionsnadel 1 im Bereich ihres Verbindungsabschnitts 1b die Dichtmembran des Medikamentenreservoirs und stellt die Fluidverbindung zur proximalen Nadelspitze her. Wenn die Nadelschutzvorrichtung am Injektionsgerät befestigt ist, drückt das Federglied 6 den Nadelschutz 7 lose gegen eine Stelle am distalen Ende des Injektionsgeräts, beispielsweise gegen einen stirnseitigen Rand des Geräts oder des Medikamentenreservoirs. Der distale Nadelschutz 5 ragt mit seinen Blockiergliedern 5a (Figur 7) in den Führungsabschnitt 4 hinein, so dass die Blockierglieder 5a in diesem Zustand keine Blockierfunktion ausüben und der Nadelschutz 5 frei gegen die Kraft des Federglieds 6 in die proximale Richtung bewegt werden kann.

Für die Injektion setzt der Verwender das Injektionsgerät mit dem distalen Ende, das nun der distale Nadelschutz 5 bildet, an der gewünschten Einstechstelle auf die Haut und bewegt das Injektionsgerät relativ zu dem Nadelschutz 5 in die distale Richtung. Der Nadelschutz 5 bewegt sich unter der Druckkraft und gegen die Kraft des Federglieds 6 in die proximale Richtung tiefer in den Führungsabschnitt 4 hinein. Gleich zu Beginn dieser Bewegung gleiten die rampenförmigen Vorsprünge 5c des Nadelschutzes 5 (Figur 7) über die angepasst rampenförmig geformten Vorsprünge 24 des Deblockierglieds 8 (Figur 8), so dass dessen Finger 23 elastisch in Richtung auf die zentrale Längsachse L gebogen werden. Bei der weitere Bewegung des Nadelschutzes 5 in die proximale Richtung gleiten die Vorsprünge 24 in axialer Richtung über die Mantelinnenfläche des Nadelschutzes 5. Während dieser Gleitbewegung sind die Finger 23 des Deblockierglieds 8 ständig nach innen elastisch gebogen und drücken mit einer rückstellenden Elastizitätskraft gegen die Mantelinnenfläche des Nadelschutzes 5. Der Nadelschutz 5 bewegt sich vorteilhafterweise vollständig in den Führungsabschnitt 4, so dass die Injektionsnadel 1 mit ihrem gesamten, über den Führungsabschnitt 4 vorstehenden Injektionsabschnitt 1a durch die Haut in das subkutane Gewebe eindringt. Ein vollständiges Einfahren des Nadelschutzes 5 in den Führungsabschnitt 4 ist im Sinne eines möglichst kurzen Nadelinjektionsabschnitts 1a vorteilhaft, aber nicht unumgänglich erforderlich.

Nach der Verabreichung des Medikaments bewegt der Verwender das Injektionsgerät von der Einstechstelle weg, so dass der Nadelschutz 5 sich unter der Einwirkung des Federglieds 6 wieder in die distale Richtung bewegt. Da das Deblockierglied 8 über die Vorsprünge 24 und die elastisch gebogenen Finger 23 kraftschlüssig mit dem Nadelschutz 5 verbunden ist, nimmt der Nadelschutz 5 das Deblockierglied 8 bei seiner Bewegung in die distale Richtung mit, so dass das Deblockierglied 8 von dem Boden 9 des Nadelhalters 2 abhebt und in Bezug auf den Nadelschutz 7 in eine Neutralposition bewegt wird. Da die Vorsprünge 24 den Nadelschutz 5 nicht mehr durchragen, bewegt sich der Nadelschutz 5 über die distale Ausgangsposition hinaus relativ zu dem Führungsabschnitt 4 in die distale Richtung. Sobald die Blockierglieder 5a des Nadelschutzes 5 die Anschläge 4c des Führungsabschnitts 4 (Figur 6) passiert haben, schnappen sie nach außen in die Ausnehmungen 4a und blockieren den Nadelschutz 5 in einer distalen Schutzposition gegen eine Bewegung zurück in die proximale Richtung. Die Eingriffsglieder 5d fahren in die Ausnehmungen 4d und halten den Nadelschutz 5 im Zusammenwirken mit den Anschlägen 4e an dem Führungsabschnitt 4. Anstatt das Deblockierglied 8 über die Vorsprünge 24 nur kraftschlüssig am Nadelschutz 5 zu halten, könnte der Eingriff auch formschlüssig gestaltet werden, indem das Deblockierglied 8 mittels der Vorsprünge 24 mit dem Nadelschutz 5 verrastet. Im Sinne einer optimalen Sicherheit ist jedoch eine frühzeitig hergestellte Verbindung vorteilhaft, wofür der beschriebene Kraftschluss ein Beispiel ist.

Für eine erneute Injektion löst der Verwender die Nadelschutzvorrichtung mit dem in seiner Schutzposition blockierten distalen Nadelschutz 5 von dem Injektionsgerät. Bei dem Lösen bewegt sich der Nadelhalter 2 relativ zu dem Injektionsgerät in die distale Richtung. Der proximale Nadelschutz 7 bewegt sich unter der Einwirkung des Federglieds 6 relativ zu dem Nadelhalter 2 in die proximale Richtung. Sobald die Vorsprünge 18 des Nadelschutzes 7 die Durchgänge 11 im Boden 9, d.h. die Durchgänge 11 des Blockierglieds des Nadelhalters 2, passiert haben, schnappen die Blockierglieder 15 des Nadelschutzes 7 elastisch nach außen vor. In diesem in der rechten Hälfte der Figur 16 gezeigten Zustand halten die Anschläge 20 der großen Vorsprünge 17 den Nadelschutz 7 gegen die Kraft des Federglieds 6 am Nadelhalter 2, und die Anschläge 19 der proximalen Vorsprünge 18 verhindern im Zusammenwirken mit den Gegenanschlägen 12 des Nadelhalters 2, das sich der Nadelschutz 7 wieder in die distale Richtung bewegen kann. Die beiden Anschläge 19 und 20 fassen den Boden 9 des Nadelhalters 2 in enger Passung zwischen sich ein, wobei das Spiel idealerweise nur so groß ist, dass die kurze Schnapp- bzw. Schwenkbewegung der Blockierglieder 15 nicht behindert wird. Grundsätzlich kann das Spiel aber auch größer sein, allerdings unter Inkaufnahme einer axialen Beweglichkeit des Nadelschutzes 7. Solch eine Beweglichkeit darf nur nicht so weit gehen, dass die distale Spitze der Injektionsnadel 1 über den Nadelschutz 7 vorragen kann.

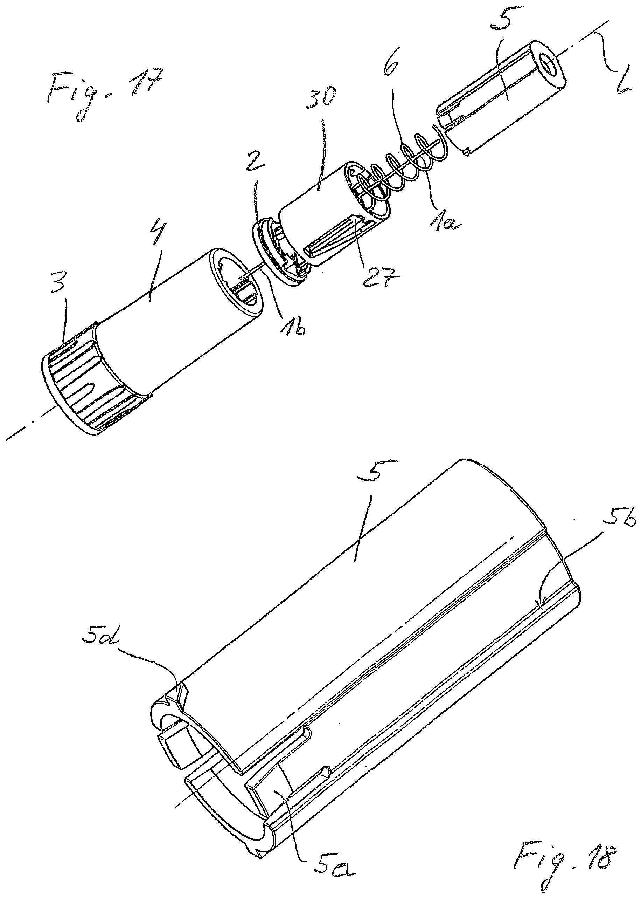

Figur 17 zeigt eine Nadelschutzvorrichtung eines zweiten Ausführungsbeispiels mit ihren längs einer zentralen Längsachse L hintereinander in der Reihenfolge der Montage aufgereihten Komponenten. Die Nadelschutzvorrichtung umfasst wieder eine Injektionsnadel 1, die wie im ersten Ausführungsbeispiel von einem Nadelhalter 2 gehalten wird, eine Befestigungs- und Führungseinrichtung 3, 4 für die Befestigung der Nadelschutzvorrichtung an dem distalen Ende eines Injektionsgeräts und die axiale Führung eines distalen Nadelschutzes 5 und ferner ein Federglied 6. Was das Zusammenwirken dieser Komponenten anbetrifft, entspricht die Nadelschutzvorrichtung dem ersten Ausführungsbeispiel. Die Befestigungs- und Führungseinrichtung 3, 4 ist mit derjenigen des ersten Ausführungsbeispiels in jeder Hinsicht identisch. Die Nadelschutzvorrichtung umfasst ferner einen distalen Nadelschutz 27 und ein Deblockierglied 30, die sich von den der Funktion nach gleichen Komponenten 7 und 8 des ersten Ausführungsbeispiels in ihrem Zusammenwirken mit den anderen Komponenten unterscheiden.

Figur 18 zeigt den distalen Nadelschutz 5 des zweiten Ausführungsbeispiels. Bei dem Nadelschutz 5 des zweiten Ausführungsbeispiels fehlen lediglich die beiden Durchbrüche mit den Vorsprüngen 5c, ansonsten entspricht der Nadelschutz 5 mit Ausnahme einer kleineren geometrischen Abweichung bei den Eingriffsgliedern 5d dem ersten Ausführungsbeispiel. Die Eingriffsglieder 5d weisen je an einer in Umfangsrichtung um die Längsachse L weisenden Seite eine Neigung zur Längsachse L auf, so dass das jeweilige Eingriffsglied 5c an der betreffenden Seite eine Rampe bildet.

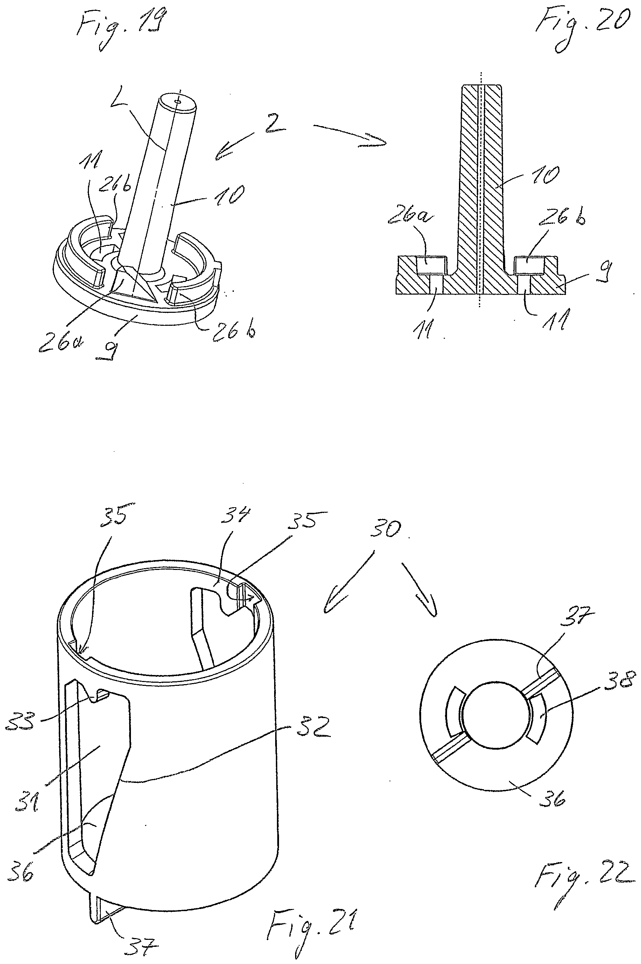

Die Figuren 19 und 20 zeigen den Nadelhalter 2 des zweiten Ausführungsbeispiels. Der Nadelhalter 2 weist wieder einen Boden 9 und einen von dem Boden 9 abragenden zentralen Haltebereich 10 für die Injektionsnadel 1 auf. Der Boden 9 ist wie bereits im ersten Ausführungsbeispiel mit zwei Durchgängen 11 versehen, durch die der Nadelschutz 27 in die proximale Richtung in seine Schutzposition einfahren kann und die ferner der Blockierung des Nadelschutzes 27 in der Schutzposition dienen. Von dem Boden 9 ragen in die distale Richtung außen neben dem Haltebereich 10 zwei in Umfangsrichtung um die Längsachse L um 180° zueinander versetzte Vorsprünge 26a ab, die je an einer Seite rampenförmig geneigt sind. Den rampenförmig geneigten Seiten der Vorsprünge 26a zugewandt ragt von dem Boden 9 ferner je ein Vorsprung 26b in die distale Richtung ab. Die Ebene des in Figur 20 dargestellten Schnitts erstreckt sich durch die Längsachse L und die Lücken zwischen je einem der Vorsprünge 26a und dem der jeweiligen Rampe zugewandten Vorsprung 26b.

Figur 21 zeigt das Deblockierglied 30 in einer perspektivischen Sicht. Figur 22 zeigt eine Draufsicht auf eine Unterseite des Deblockierglieds 30, d.h. eine Ansicht in die distale Richtung. Das Deblockierglied 30 ist hohlzylindrisch. In dem Mantel des Deblockierglieds 30 sind in Umfangsrichtung um 180° zueinander versetzt zwei Ausnehmungen 31 geformt, die den Mantel durchbrechen und je an einer Seite eine in Umfangsrichtung geneigte Führungsbahn 32 für eines der Eingriffsglieder 5d des distalen Nadelschutzes 5 bilden. Von einem distalen Rand des Deblockierglieds 30 ragt je ein Vorsprung 33 in die jeweilige Ausnehmung 31. Die beiden Ausnehmungen 31 sind von dem Mantel des Deblockierglieds 30 umlaufend umrahmt, wobei distal vor den Führungsbahnen 32 an der Innenseite des Mantels je eine Ausnehmung 35 geformt ist, die sich von dem distalen Ende des Deblockierglieds 30 durchgehend bis in die jeweilige Ausnehmung 31 erstreckt. Bei der Montage der Nadelschutzvorrichtung wird das Deblockierglied 30 mit seinen beiden Ausnehmungen 35 über die Eingriffsglieder 5d des Nadelschutzes 5 bewegt, so dass die Eingriffsglieder 5d in die jeweils zugeordnete Ausnehmung 31 einfahren. Anschließend werden die Eingriffsglieder 5d durch Drehen des Deblockierglieds 30 ebenfalls noch im Verlaufe der Montage in Umfangsrichtung hinter den jeweiligen Vorsprung 33 in den Umfangsbereich 34 bewegt, so dass die Eingriffsglieder 5d den distalen Rand des Deblockierglieds 30 in dessen Umfangsbereichen 34 hintergreifen und der Nadelschutz 5 dadurch gegen die Kraft des Federglieds 6 an dem Führungsabschnitt 4 gehalten wird. Das Deblockierglied 30 ist in die distale Richtung auf Anschlag gegen den Führungsabschnitt 4, ist aber axial zwischen diesem Anschlag und dem Boden 9 des Nadelhalters 2 hin und her beweglich und ferner relativ zu dem Nadelhalter 2 um die Längsachse L drehbar.

Das Deblockierglied 30 weist an seiner Unterseite, am proximalen Ende seines Mantels einen nach radial innen ragenden ringförmigen Boden 36 und von dem Boden 36 in die proximale Richtung abragende Eingriffsglieder 37 auf, insgesamt zwei Eingriffsglieder 37, die um 180° in Umfangsrichtung zueinander versetzt sind. In dem Boden 36 sind zwei um 180° in Umfangsrichtung zueinander versetzte Durchgänge 38 für den proximalen Nadelschutz 27 geformt.

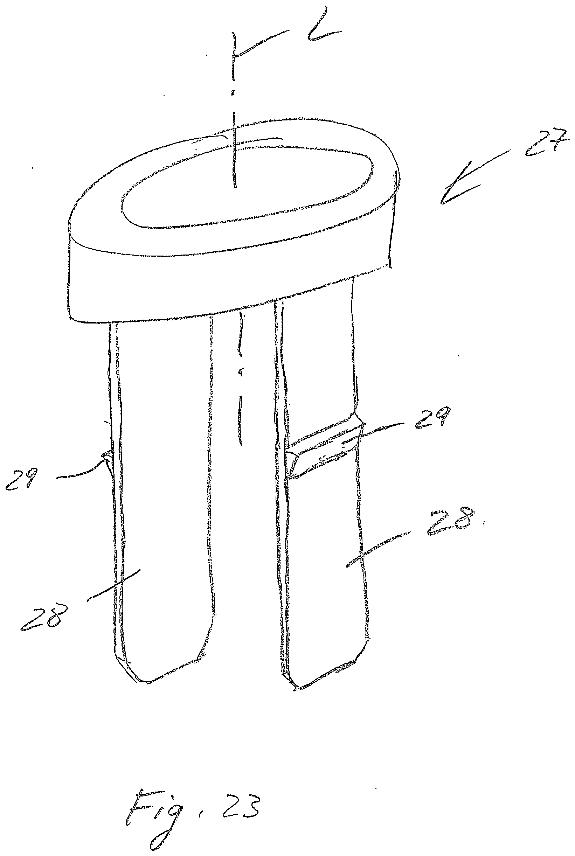

Figur 23 zeigt den proximalen Nadelschutz 27. Der Nadelschutz 27 weist an einem distalen Ende eine ringförmige Basis und von der Basis in die proximale Richtung abragende, elastisch in Richtung auf die Längsachse L biegbare Beine oder Finger auf, die Blockierglieder 28 des Nadelschutzes 27 bilden. An der Außenseite der Blockierglieder 28 ist je ein Vorsprung 29 geformt. Die Vorsprünge 29 verjüngen sich pfeilförmig in die proximale Richtung und bilden wie bereits die Vorsprünge 17 des ersten Ausführungsbeispiels je einen in die distale Richtung weisenden Anschlag des Nadelschutzes 27. Diesbezüglich wird auf die Ausführung zum ersten Ausführungsbeispiel verwiesen.

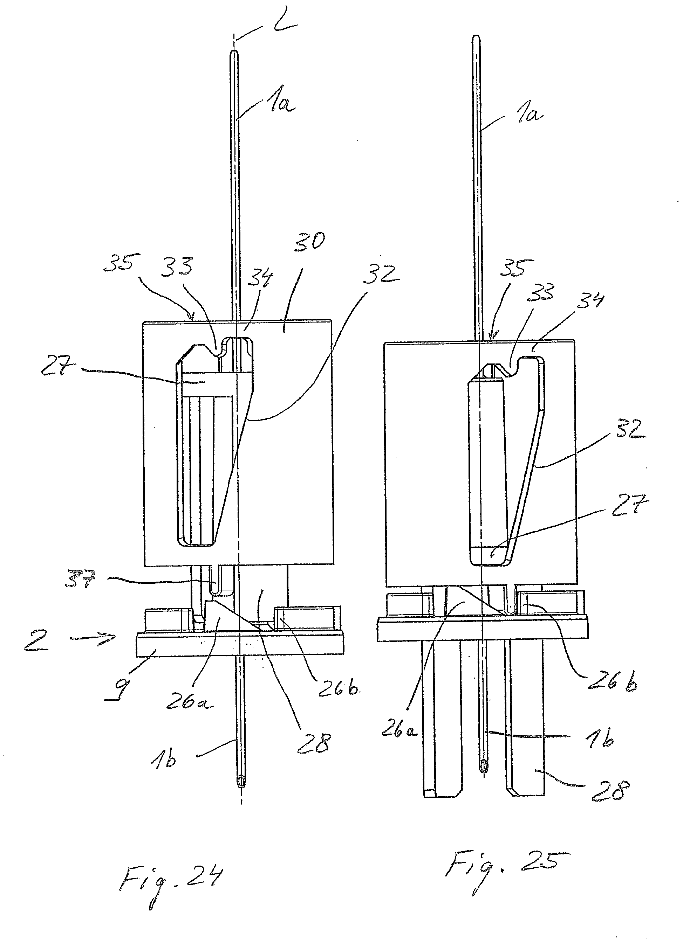

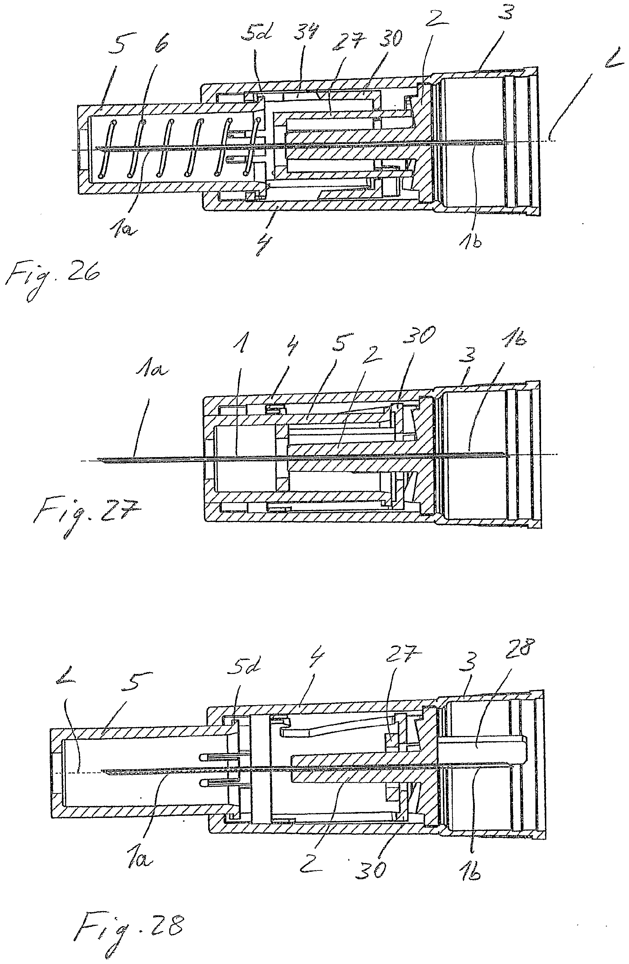

Figur 24 zeigt die bei der Blockierung des Nadelschutzes 27 zusammenwirkenden Komponenten der Blockiereinrichtung des zweiten Ausführungsbeispiels, wobei der Nadelschutz 27 eine distale Ausgangsposition einnimmt, die gleichzeitig auch der Freigabeposition des Nadelschutzes 27 entspricht. Der Nadelschutz 27 steht im zweiten Ausführungsbeispiel in der Ausgangsposition bereits vollständig hinter dem Verbindungsabschnitt 1b der Injektionsnadel 1 in die distale Richtung zurück. Er steht auch hinter dem Boden 9 des Nadelhalters 2 zurück, d.h. das Federglied 6 drückt den Nadelschutz 27 in dessen Ausgangsposition in die proximale Richtung gegen den Boden 9. Die Blockierglieder 28 durchragen die Durchgänge 38 des Deblockierglieds 30 (Figur 22). Im Ausgangszustand der Nadelschutzvorrichtung sind die Durchgänge 38 und die Durchgänge 11 des Nadelhalters 2 in Umfangsrichtung zueinander versetzt. Die Eingriffsglieder 37 des Deblockierglieds 30 sind den rampenförmigen Seiten der Vorsprünge 26a des Nadelhalters 2 axial zugewandt. Das Federglied 6 hält das Deblockierglied 30 über den Nadelschutz 5 in der in Figur 24 gezeigten distalen Ausgangsposition, indem der Nadelschutz 5 mittels seiner Eingriffsglieder 5d das Deblockierglied 30 in den Umfangsbereichen 34 hintergreift und in die distale Ausgangsposition zieht und dadurch auf Anschlag gegen den Führungsabschnitt 4 drückt. Diesen Ausgangszustand zeigt auch Figur 26.

Figur 25 zeigt die Komponenten der Blockiereinrichtung im Endzustand nach Gebrauch der Nadelschutzvorrichtung und mit dem in seiner proximalen Schutzposition befindlichen Nadelschutz 27. Figur 28 zeigt die Nadelschutzvorrichtung insgesamt ebenfalls in diesem Endzustand.

Nachfolgend wird die Funktionsweise der Nadelschutzvorrichtung des zweiten Ausführungsbeispiels anhand der Figuren 26 bis 28, aber unter Verweis auf die weiteren Figuren 17 bis 25 und insbesondere auf die Figuren 24 und 25 beschrieben.

Die Nadelschutzvorrichtung wird in dem in den Figuren 24 und 26 dargestellten Ausgangszustand mit dem Injektionsgerät verbunden, beispielsweise verschraubt oder verklippt. Dabei durchdringt der Nadelverbindungsabschnitt 1b die Dichtmembran des Medikamentenreservoirs. Eine Bewegung des Nadelschutzes 27 findet bei der Befestigung noch nicht statt.

Anschließend sticht der Verwender die Injektionsnadel 1 an der gewünschten Einstechstelle durch die Haut in das subkutane Gewebe. Dabei bewegt sich der Nadelschutz 5 relativ zu dem Nadelhalter 2 in die proximale Richtung, so dass umgekehrt der Nadelinjektionsabschnitt 1a vorsticht. Figur 27 zeigt die Nadelschutzvorrichtung im eingestochenen Zustand während der Verabreichung des Medikaments.

Bei der Bewegung des Nadelschutzes 5 in die proximale Richtung fahren die Eingriffsglieder 5d die jeweils zugehörige Führungsbahn 32 des Deblockierglieds 30 ab. Die Neigung der Führungsbahn 32 ist so gewählt, dass eine Hemmung nicht auftreten kann. Durch diesen Führungseingriff wird das Deblockierglied 30 aus seiner in Figur 24 gezeigten Drehwinkelposition, der Deblockierposition, in die Drehwinkelposition (Neutralposition) der Figur 25 gedreht. Der Drehbewegung ist eine axiale Translationsbewegung überlagert, bei der die Eingriffsglieder 37 des Deblockierglieds 30 auf der rampenförmigen Seite des jeweils zugeordneten Vorsprungs 26a gleiten. Die Translations- und Rotationsbewegung wird durch Anschlagkontakt der Eingriffsglieder 37 und der Vorsprünge 26b begrenzt. Das Deblockierglied 30 nimmt den Nadelschutz 27 bei der Drehbewegung mit, da die Blockierglieder 28 die Durchgänge 38 durchgreifen. Sobald das Deblockierglied 30 seine in Figur 25 dargestellte Neutralposition erreicht hat, überlappen die Durchgäng 38 des Deblockierglieds 30 (Figur 22) die Durchgänge 11 des Nadelhalters 2 (Figur 19 und 20) so weit, dass die Blockierglieder 28 des Nadelschutzes 27 unter der Kraft des Federglieds 6 die Durchgänge 11 passieren können. Die Blockierglieder 28 werden von dem Federglied 6 bis gegen eine Stelle am distalen Ende des Injektionsgeräts, beispielsweise eine distale Stelle des Geräts selbst oder des Medikamentenreservoirs, gedrückt. Die Blockierglieder 28 verhindern nach dem Einfahren in die Durchgänge 11 des Nadelhalters 2, dass sich das Deblockierglied 30 wieder in seine Deblockierposition (Figur 24) zurückdrehen kann.
Wenn die Injektionsnadel 1 aus dem Gewebe gezogen und dadurch der Nadelschutz 5 entlastet wird, bewegt das Federglied 6 den Nadelschutz 5 in die distale Richtung. Die Position der beiden Vorsprünge 33 ist relativ zu der jeweils zugewandten Führungsbahn 32 in Umfangsrichtung so gewählt, dass die Eingriffsglieder 5d bei der Bewegung des Nadelschutzes 5 in die distale Richtung in die Ausnehmungen 35 einfahren, wodurch der Nadelschutz 5 schließlich von dem Deblockierglied 30 freikommt und die Blockierglieder 5a wie im ersten Ausführungsbeispiel in die Ausnehmungen 4a des Führungsabschnitts 4 einfahren können (Figur 6) und durch Blockiereingriff mit den Anschlägen 4c den Nadelschutz 5 in seiner distalen Schutzposition gegen eine Bewegung in die proximale Richtung blockieren. Die Eingriffsglieder 5d verhindern wie im ersten Ausführungsbeispiel im Zusammenwirken mit den Anschlägen 4e (Figur 6), dass der Nadelschutz 5 aus dem Führungsabschnitt 4 in die distale Richtung vollständig herausfahren kann.

Die Nadelschutzvorrichtung wird mit dem blockierten Nadelschutz 5 von dem Injektionsgerät gelöst. Der proximale Nadelschutz 27 fährt unter der Einwirkung des Federglieds 6 in die in Figur 25 und Figur 28 dargestellte Schutzposition in die proximale Richtung relativ zu dem Nadelhalter 2 aus. In der Schutzposition ragen die Blockierglieder 28 über die Spitze des Nadelverbindungsabschnitts 1b in die proximale Richtung vor. Der Nadelschutz 27 wird durch einen Blockiereingriff zwischen seinen Vorsprüngen 29 und dem Boden 9 des Nadelhalters 2 gegen eine Bewegung zurück in die distale Richtung blockiert. Der Nadelschutz 27 stützt sich über seine seine ringförmige Basis in die proximale Richtung an dem Boden 9 des Nadelhalters 2 ab.

### Bezugszeichen:

- 1: Injektionsnadel
- 1a: Nadelinjektionsabschnitt
- 1b: Nadelverbindungsabschnitt
- 2: Nadelhalter
- 3: Befestigungsabschnitt
- 4: Führungsabschnitt
- 4a: Ausnehmung
- 4b: Führung
- 4c: Anschlag
- 4d: Ausnehmung
- 4e: Anschlag
- 5: distaler Nadelschutz
- 5a: Blockierglied
- 5b: Führung
- 5c: Vorsprung
- 5d: Eingriffsglied
- 6: Federglied
- 7: proximaler Nadelschutz
- 8: Deblockierglied
- 9: Boden, erstes Blockierglied 10 Haltebereich
- 11: Durchgang
- 12: Gegenanschlag
- 13: Gegenanschlag
- 14: Basis
- 15: Finger, zweites Blockierglied
- 16: Vorsprung
- 17: Vorsprung
- 18: Vorsprung
- 19: Anschlag
- 20: Anschlag
- 21: Federabstützung
- 22: Basis
- 23: Finger
- 24: Vorsprung
- 25: Führungsbahn
- 26a: Vorsprung
- 26b: Vorsprung
- 27: Proximaler Nadelschutz
- 28: zweites Blockierglied
- 29: Vorsprung
- 30: Deblockierglied
- 31: Ausnehmung
- 32: Führungsbahn
- 33: Vorsprung
- 34: Umfangsbereich
- 35: Ausnehmung
- 36: Boden
- 37: Eingriffsglied
- 38: Durchgang

- L: Längsrichtung, Längsachse

## Patentansprüche

1. Nadelschutzvorrichtung, die an einem Injektionsgerät lösbar befestigt oder befestigbar ist, die Nadelschutzvorrichtung umfassend:
a) eine Injektionsnadel (1) mit einem sich bis zu einem distalen Ende der Injektionsnadel (1) Ende der Injektionsnadel (1) erstreckenden Nadelverbindungsabschnitt (1b),
b) einen Nadelhalter (2), von dem der Nadelinjektionsabschnitt (1a) in die distale Richtung und der Nadelverbindungsabschnitt (1b) in die proximale Richtung vorragen,
c) einen mit dem Nadelhalter (2) beweglich verbundenen distalen Nadelschutz (5), der aus einer Freigabeposition in die distale Richtung bis in eine Schutzposition bewegbar ist, wobei der distale Nadelschutz (5) in der Freigabeposition hinter dem Nadelinjektionsabschnitt (1a) zurücksteht und in der Schutzposition den Nadelinjektionsabschnitt (1a) einschliesslich des distalen Endes der Injektionsnadel (1) überlappt,
d) einen mit dem Nadelhalter (2) beweglich verbundenen proximalen Nadelschutz (7; 27) der aus einer Freigabeposition in die proximale Richtung bis in eine Schutzposition bewegbar ist, wobei der proximale Nadelschutz (7; 27) in der Freigabeposition hinter dem Nadelverbindungsabschnitt (1b) zurücksteht und in der Schutzposition den Nadelverbindungsabschnitt (1 b) einschliesslich des proximalen Endes der Injektionsnadel (1) überlappt,
e) eine Blockiereinrichtung (9, 15, 8; 9, 28, 30), die eine Bewegung des proximalen Nadelschutzes (7; 27) aus seiner Schutzposition in seine Freigabeposition blockiert,
f) wobei die Blockiereinrichtung (9, 15, 8; 9, 28, 30) ein von dem Nadelhalter (2) gebildetes oder mit dem Nadelhalter (2) verbundenes erstes Blockierglied (9) und ein von dem proximalen Nadelschutz (7; 27) gebildetes oder mit dem proximalen Nadelschutz verbundenes zweites Blockierglied (15; 28) umfasst, dass das zweite Blockierglied (15, 28) bei der Bewegung des proximalen Nadelschutzes (7; 27) in die proximale Richtung mit dem ersten Blockierglied (9) in einen Blockiereingriff gelangt, der eine Bewegung des proximalen Nadelschutzes (7; 27) zurück in die distale Richtung verhindert, wenn der proximale Nadelschutz (7; 27) seine Schutzposition erreicht hat, und dass zumindest bei in Schutzposition befindlichem proximalen Nadelschutz (7; 27) der Nadelhalter (2) relativ zu dem ersten Blockierglied (9) nicht in die proximale Richtung und der proximale Nadelschutz (7; 27) relativ zu dem zweiten Blockierglied (15; 28) nicht in die distale Richtung bewegt werden können,
g) **dadurch gekennzeichnet, dass** die Blockiereinrichtung ein Deblockierglied (8; 30) umfasst, das relativ zu dem proximalen Nadelschutz (7; 27), vorzugsweise auch relativ zu dem Nadelhalter (2), aus einer Deblockierposition, in der es den Blockiereingriff verhindert, in eine Neutralposition beweglich ist, in der es den Blockiereingriff zulässt.

2. Nadelschutzvorrichtung nach Anspruch 1, ferner umfassend eine Befestigungseinrichtung (3) für eine lösbare Befestigung der Nadelschutzvorrichtung an dem Injektionsgerät, wobei der proximale Nadelschutz (7; 27) dem Nadelverbindungsabschnitt (1b) näher als die Befestigungseinrichtung (3) ist.

3. Nadelschutzvorrichtung nach dem vorhergehenden Anspruch, **dadurch gekennzeichnet, dass** die Befestigungseinrichtung (3) den Nadelverbindungsabschnitt (1b) überlappt, vorzugsweise einschiesslich des proximalen Endes der Injektionsnadel. (1).

4. Nadelschutzvorrichtung nach einem der zwei vorhergehenden Ansprüche, **dadurch gekennzeichnet, dass** die Befestigungseinrichtung (3) den Nadelverbindungsabschnitt (1b) und den in seiner Schutzposition befindlichen proximalen Nadelschutz (7; 27) umgibt.

5. Nadelschutzvorrichtung nach einem der vorhergehenden Ansprüche, ferner umfassend ein Federglied (6), das den proximalen Nadelschutz (7; 27) in die proximale Richtung mit einer Federkraft beaufschlagt, um den proximalen Nadelschutz (7; 27) in seine Schutzposition zu bewegen.

6. Nadelschutzvorrichtung nach dem vorhergehenden Anspruch, **dadurch gekennzeichnet, dass** das Federglied (6) den distalen Nadelschutz (5) in die distale Richtung mit einer Federkraft beaufschlagt, um den distalen Nadelschutz (5) in seine Schutzposition zu bewegen.

7. Nadelschutzvorrichtung nach einem der vorhergehenden Ansprüche, **dadurch gekennzeichnet, dass** der proximale Nadelschutz (7; 27) den Nadelhalter (2) zumindest in der Freigabeposition von proximal nach distal durchragt.

8. Nadelschutzvorrichtung nach einem der vorhergehenden Anspruch, **dadurch gekennzeichnet, dass** das erste Blockierglied (9) einen in die proximale Richtung weisenden Anschlag (12) und das zweite Blockierglied (15; 28) einen in die distale Richtung weisenden Anschlag (19) bilden und dass die Anschläge (12, 19) bei der Bewegung des proximalen Nadelschutzes (7; 27) in die proximale Richtung quer zu der Längsrichtung (L) der Injektionsnadel (1) in eine Überlappung gelangen, die eine Bewegung des proximalen Nadelschutzes (7; 27) zurück in die Freigabeposition verhindert.

9. Nadelschutzvorrichtung nach einem der vorhergehenden Ansprüche, **dadurch gekennzeichnet, dass** der Nadelhalter (2) seitlich neben der Injektionsnadel (1) einen von proximal nach distal erstreckten Durchgang (11) aufweist, den das zweite Blockierglied (15; 28) in der Freigabeposition oder der Schutzposition des proximalen Nadelschutzes (7; 27) durchragt.

10. Nadelschutzvorrichtung nach dem vorhergehenden Anspruch, **dadurch gekennzeichnet, dass** eine in die proximale Richtung weisende Schulter, die ein proximales Ende des Durchgangs (11) bildet oder in dem Durchgang geformt ist, einen Anschlag (12) des ersten Blockierglieds (19) bildet.

11. Nadelschutzvorrichtung nach einem der vorhergehenden Ansprüche, **dadurch gekennzeichnet, dass** sich das zweite Blockierglied (15; 28) fingerförmig von proximal nach distal erstreckt und mit einer Schulter versehen ist, die einen Anschlag (19) für das erste Blockierglied (9) bildet.

12. Nadelschutzvorrichtung nach einem der vorhergehenden Ansprüche, **dadurch gekennzeichnet, dass** wenigstens eines der Blockierglieder (9, 15) gegen eine rückstellende Federkraft, die vorzugsweise auf einer Formelastizität des wenigstens einen der Blockierglieder (9, 15) beruht, quer zu einer Längsrichtung (L) der Injektionsnadel (1) beweglich ist.

13. Nadelschutzvorrichtung nach einem der vorhergehenden Ansprüche, **dadurch gekennzeichnet, dass** der proximale Nadelschutz (7; 27) an einem proximalen oder distalen Ende einen Bogen (14; 22) um den Nadelverbindungsabschnitt (1 b) bildet und das zweite Blockierglied (15; 28) von dem Bogen (14; 22) fingerförmig nach distal oder proximal abragt.

14. Nadelschutzvorrichtung nach einem der vorhergehenden Anspruch, **dadurch gekennzeichnet, dass** das zweite Blockierglied (15; 28) in der Freigabeposition des proximalen Nadelschutzes (7; 27) oder einer Bewegung des proximalen Nadelschutzes (7) in die Freigabeposition mit dem in der Deblockierposition befindlichen Deblockierglied (15; 28) in einem Kontakt ist, der den Blockiereingriff verhindert.

15. Nadelschutzvorrichtung nach einem der vorhergehenden Ansprüche, **dadurch gekennzeichnet, dass** das Deblockierglied (8; 30) mit dem distalen Nadelschutz gekoppelt ist oder bei der Bewegung in die proximale Richtung oder bei der Bewegung in die distale Richtung automatisch mit dem distalen Nadelschutz koppelbar ist.

16. Nadelschutzvorrichtung nach einem der vorhergehenden Ansprüche, **dadurch gekennzeichnet, dass** der distale Nadelschutz (5) aus einer distalen Ausgangsposition in die proximale Richtung bis in seine Freigabeposition beweglich ist und bei der Bewegung in die proximale Richtung oder in der Freigabeposition oder der Bewegung in die distale Richtung mit dem Deblockierglied (8; 39) in einen Eingriff gelangt, der die Bewegung des Deblockierglieds (8; 30) in die Neutralposition bewirkt.

17. Nadelschutzvorrichtung nach dem Anspruch 15 und 16, **dadurch gekennzeichnet, dass** die Koppelung als Mitnahmeeingriff ausgebildet ist, wobei der distale Nadelschutz bei seiner Bewegung in die distale Richtung das Deblockierglied bis in die Neutralposition mitnimmt, oder als Kulissenführung ausgebildet ist, wobei die Kulissenführung die Bewegung in die proximale Richtung oder die Bewegung in die distale Richtung des distalen Nadelschutzes in die Bewegung des Deblockierglieds aus der Deblockierposition in die Neutralposition umwandelt.

## Claims

1. Needle protection device, which is disconnectably fitted or can be fitted to an injection unit, with the needle protection device comprising:
a) an injection needle (1) with a needle connection section (1b) extending to a distal end of the injection needle (1) end of the injection needle (1),
b) a needle holder (2), from which the needle injection section (1a) projects in the distal direction and the needle connection section (1b) projects in the proximal direction,
c) a distal needle protection (5), moveable connected with the needle holder (2), which can be moved from a release position in the distal direction up to a protected position, wherein the distal needle protection (5) stands back behind the needle injection section (1a) in the release position and overlaps the needle injection section (1a) including the distal end of the injection needle (1) in the protected position,
d) a proximal needle protection (7; 27), moveably connected with the needle holder (2), which can be moved from a release position in the proximal direction up to a protected position, wherein the proximal needle protection (7; 27) stands back behind and overlaps the needle connection section (1b) including the proximal end of the injection needle (1) in the release position,
e) a block means (9, 15, 8; 9, 28, 30), which blocks a movement of the proximal needle protection (7, 27) from its protected position into its release position,
f) wherein the block means (9, 15, 8, 9, 28, 30) comprises a first blocking member (9) formed by the needle holder (2) or connected with the needle holder (2), and a second blocking member (15; 28) formed by the proximal needle protection (7; 27) or connected with the proximal needle protection, that the second blocking member (15, 28) arrives in a blocking engagement with the first blocking member (9) during the movement of proximal needle protection (7; 27) in the proximal direction, which prevents a movement of the proximal needle protection (7; 27) back in the distal direction when the proximal needle protection (7; 27) has reached its protection position, and that the needle holder (2) cannot be moved in the proximal direction relative to the first blocking member (9), and the proximal needle protection (7; 27) cannot be moved in the distal direction relative to the second blocking member (15; 28) at least when the proximal needle protection (7; 27) is in the protection position,
g) **characterised in that** the blocking means comprises a deblocking member (8; 30), which can be moved from a deblocking position, in which it prevents the deblocking action, into a neutral position, in which it allows the blocking action, relative to the proximal needle protection (7; 27), preferably also relative to the needle holder (2).

2. Needle protection device according to claim 1, further comprising a fitting means (3) for a disconnectable fitting of the needle protection device on an injection unit, wherein the proximal needle protection (7; 27) is closer to the needle connection section (1b) than the fitting means (3).

3. Needle protection device according to the preceding claims, **characterised in that** the fitting means (3) overlaps the needle connection section (1b), preferably including the proximal end of the injection needle (1).

4. Needle protection device according to one of the two preceding claims, **characterised in that** the fitting means (3) surrounds the needle connection section (1b) and the proximal needle protection (7; 27) in its protected position.

5. Needle protection device according to one of the preceding claims, further comprising a spring member (6) that applies a spring force to the proximal needle protection (7; 27) in the proximal direction, for moving the proximal needle protection (7; 27) into its protected position.

6. Needle protection device according to the preceding claim, **characterised in that** the spring member (6) applies a spring force to the distal needle protection (5) in the distal direction, for moving the distal needle protection (5) into its protected position.

7. Needle protection device according to one of the preceding claims, **characterised in that** the proximal needle protection (7; 27) projects through the needle holder (2) from proximal to distal at least in the release position.

8. Needle protection device according to one of the preceding claims, **characterised in that** the first blocking member (9) forms a stop (12) facing in the proximal direction, and the second blocking member (15; 28) forms a stop (19) facing in the distal direction, and **in that** the stops (12, 19) arrive in an overlapping in the proximal direction transverse to the longitudinal direction (L) of the injection needle (1) upon movement of the proximal needle protection (7; 27), which prevents a movement of the proximal needle protection (7; 27) back in the release position.

9. Needle protection device according to one of the preceding claims, **characterised in that** the needle holder (2) comprises a passage (11) extending from proximal to distal at the side next to the injection needle (1), through which the second blocking member (15; 28) projects in the release position or the protected position of the proximal needle protection (7; 27).

10. Needle protection device according to the preceding claim, **characterised in that** a stop facing the proximal direction, which forms a proximal end of the passage (11) or is formed in the passage, forms a stop (12) of the first blocking member (19).

11. Needle protection device according to one of the preceding claim, **characterised in that** the second blocking member (15; 28) extends finger-shaped from proximal to distal and is equipped with a shoulder that forms a stop (19) for the first blocking member (9).

12. Needle protection device according to one of the preceding claim, **characterised in that** at least one of the blocking members (9, 15) can be moved against a returning spring force, which preferably bases on a shape elasticity of the at least one of the blocking members (9, 15), transverse to a longitudinal direction (L) of the injection needle (1).

13. Needle protection device according to one of the preceding claim, **characterised in that** the proximal needle protection (7; 27) forms an arc (14; 22) around the needle connection section (1b) at a proximal or distal end, and the second blocking member (15; 28) projects finger-shaped in a distal or proximal direction from the arc (14; 22).

14. Needle protection device according to one of the preceding claim, **characterised in that** the second blocking member (15; 28) is in a contact that prevents blocking engagement in the release position of the proximal needle protection (7; 27) or during a movement of the proximal needle protection (7) in the release position with the deblocking member (15; 28) located in the deblocking member (15; 28).

15. Needle protection device according to one of the preceding claim, **characterised in that** the deblocking member (8; 30) is coupled with the distal needle protection or during the movement in the proximal direction, or can be automatically coupled with the distal needle protection during the movement in the distal direction.

16. Needle protection device according to one of the preceding claim, **characterised in that** the distal needle protection (5) can be moved from a distal starting position in the proximal direction up to its release position, and arrives in an engagement that effects the movement of the deblocking members (8; 30) into the neutral position during its movement in the proximal direction or in the release position, or the movement in the distal direction with the deblocking member (8; 39).

17. Needle protection device according to claim 15 and 16, **characterised in that** the coupling is designed as a follower engagement, wherein the distal needle protection takes the deblocking members with it up to the neutral position during its movement in the distal direction, or is designed as a slotted guide system, wherein the slotted guide system converts the movement in the proximal direction or the movement in the distal direction of the distal needle protection into the movement of the deblocking members out of the deblocking position into the neutral position.

## Revendications

1. Dispositif de protection d'aiguille, qui est fixé ou peut être fixé de manière amovible au niveau d'un appareil d'injection, comprenant :
a) une aiguille d'injection (1) avec un tronçon de liaison d'aiguille (1b) s'étendant jusqu'à une extrémité distale de l'aiguille d'injection (1) extrémité de l'aiguille d'injection (1),
b) un porte-aiguille (2), duquel le tronçon d'injection d'aiguille (1a) dépasse dans la direction distale et duquel le tronçon de liaison d'aiguille (1b) dépasse dans la direction proximale,
c) une protection d'aiguille (5) distale reliée de manière mobile au porte-aiguille (2), laquelle peut être déplacée hors d'une position dégagée dans la direction distale jusque dans une position de protection, dans lequel la protection d'aiguille (5) distale est en retrait, dans la position dégagée, derrière le tronçon d'injection d'aiguille (1a) et chevauche, dans la position de protection, le tronçon d'injection d'aiguille (1a) y compris l'extrémité distale de l'aiguille d'injection (1),
d) une protection d'aiguille (7 ; 27) proximale reliée de manière mobile au porte-aiguille (2), laquelle peut être déplacée depuis une position dégagée dans la direction proximale jusque dans une position de protection, dans lequel la protection d'aiguille (7 ; 27) proximale est en retrait, dans la position dégagée, derrière le tronçon de liaison d'aiguille (1b) et chevauche, dans la position de protection, le tronçon de liaison d'aiguille (1b) y compris l'extrémité proximale de l'aiguille d'injection (1),
e) un système de blocage (9, 15, 8 ; 9, 28, 30), qui bloque un déplacement de la protection d'aiguille (7 ; 27) proximale hors de sa position de protection dans sa position dégagée,
f) dans lequel le système de blocage (9, 15, 8 ; 9, 28, 30) comprend un premier organe de blocage (9) formé par le porte-aiguille (2) ou relié au porte-aiguille (2) et un deuxième organe de blocage (15 ; 28) formé par la protection d'aiguille (7 ; 27) proximale ou relié à la protection d'aiguille proximale, dans lequel le deuxième organe de blocage (15 ; 28) parvient, lors du déplacement de la protection d'aiguille (7 ; 27) proximale dans la direction proximale, dans une prise par blocage avec le premier organe de blocage (9), laquelle prise par blocage empêche un déplacement de retour de la protection d'aiguille (7 ; 27) proximale dans la direction distale, quand la protection d'aiguille (7 ; 27) proximale a atteint sa position de protection, et dans lequel le porte-aiguille (2) ne peut être déplacé dans la direction proximale par rapport au premier organe de blocage (9) et la protection d'aiguille (7 ; 27) proximale ne peut être déplacée dans la direction distale par rapport au deuxième organe de blocage (15 ; 28) au moins lorsque la protection d'aiguille (7 ; 27) proximale se trouve en position de protection,
g) **caractérisé en ce que** le système de blocage comprend un organe de déblocage (8 ; 30), qui est mobile, par rapport à la protection d'aiguille (7 ; 27) proximale, de préférence également par rapport au porte-aiguille (2), de manière à sortir d'une position de déblocage, dans laquelle il empêche la prise par blocage, pour aller dans une position neutre, dans laquelle il autorise la prise par blocage.

2. Dispositif de protection d'aiguille selon la revendication 1, comprenant en outre un système de fixation (3) pour une fixation amovible du dispositif de protection d'aiguille au niveau de l'appareil d'injection, dans lequel la protection d'aiguille (7 ; 27) proximale est plus proche du tronçon de liaison d'aiguille (1b) que le système de fixation (3).

3. Dispositif de protection d'aiguille selon la revendication précédente, **caractérisé en ce que** le système de fixation (3) chevauche le tronçon de liaison d'aiguille (1b), de préférence y compris l'extrémité proximale de l'aiguille d'injection (1).

4. Dispositif de protection d'aiguille selon l'une quelconque des deux revendications précédentes, **caractérisé en ce que** le système de fixation (3) entoure le tronçon de liaison d'aiguille (1b) et la protection d'aiguille (7 ; 27) proximale se trouvant dans sa position de protection.

5. Dispositif de protection d'aiguille selon l'une quelconque des revendications précédentes, comprenant en outre un organe à ressorts (6), qui soumet la protection d'aiguille (7 ; 27) proximale dans la direction proximale à l'action d'une force de ressort afin de déplacer la protection d'aiguille (7 ; 27) proximale dans sa position de protection.

6. Dispositif de protection d'aiguille selon la revendication précédente, **caractérisé en ce que** l'organe à ressorts (6) soumet la protection d'aiguille (5) distale dans la direction distale à l'action d'une force de ressort afin de déplacer la protection d'aiguille (5) distale dans sa position de protection.

7. Dispositif de protection d'aiguille selon l'une quelconque des revendications précédentes, **caractérisé en ce que** la protection d'aiguille (7 ; 27) proximale traverse le porte-aiguille (2) au moins dans la position dégagée du côté proximal vers le côté distal.

8. Dispositif de protection d'aiguille selon l'une quelconque des revendications précédentes, **caractérisé en ce que** le premier organe de blocage (9) forme une butée (12) pointant dans la direction proximale et le deuxième organe de blocage (15 ; 28) forme une butée (19) pointant dans la direction distale, et **en ce que** les butées (12, 19) parviennent, lors du déplacement de la protection d'aiguille (7 ; 27) proximale dans la direction proximale, de manière transversale par rapport à la direction longitudinale (L) de l'aiguille d'injection (1) en un chevauchement, qui empêche un déplacement de retour de la protection d'aiguille (7 ; 27) proximale dans la position dégagée.

9. Dispositif de protection d'aiguille selon l'une quelconque des revendications précédentes, **caractérisé en ce que** le porte-outil (2) présente, latéralement à côté de l'aiguille d'injection (1), un passage (11) s'étendant du côté proximal vers le côté distal, que le deuxième organe de blocage (15 ; 28) traverse dans la position dégagée ou dans la position de protection de la protection d'aiguille (7 ; 27) proximale.

10. Dispositif de protection d'aiguille selon la revendication précédente, **caractérisé en ce qu'**un épaulement pointant dans la direction proximale, qui forme une extrémité proximale du passage (11) ou qui est formé dans le passage, forme une butée (12) du premier organe de blocage (19).

11. Dispositif de protection d'aiguille selon l'une quelconque des revendications précédentes, **caractérisé en ce que** le deuxième organe de blocage (15 ; 28) s'étend sous la forme d'un doigt du côté proximal vers le côté distal et est pourvu d'un épaulement, qui forme une butée (19) pour le premier organe de blocage (9).

12. Dispositif de protection d'aiguille selon l'une quelconque des revendications précédentes, **caractérisé en ce qu'**au moins un des organes de blocage (9, 15) est mobile de manière transversale par rapport à une direction longitudinale (L) de l'aiguille d'injection (1) à l'encontre de la force de ressort de rappel, qui repose de préférence sur une élasticité de forme de l'au moins un organe de blocage des organes de blocage (9, 15).

13. Dispositif de protection d'aiguille selon l'une quelconque des revendications précédentes, **caractérisé en ce que** la protection d'aiguille (7 ; 27) proximale forme, au niveau d'une extrémité proximale ou distale, un arc (14 ; 22) autour du tronçon de liaison d'aiguille (1b), et **en ce que** le deuxième organe de blocage (15 ; 28) dépasse de l'arc (14 ; 22) sous la forme d'un doigt vers le côté distal ou proximal.

14. Dispositif de protection d'aiguille selon l'une quelconque des revendications précédentes, **caractérisé en ce que** le deuxième organe de blocage (15 ; 28) est en contact, dans la position dégagée de la protection d'aiguille (7 ; 27) proximale ou dans un déplacement de la protection d'aiguille (7) proximale dans la position dégagée, avec l'organe de déblocage (15 ; 28) se trouvant dans la position de déblocage, lequel contact empêche la prise par blocage.

15. Dispositif de protection d'aiguille selon l'une quelconque des revendications précédentes, **caractérisé en ce que** l'organe de déblocage (8 ; 30) est couplé à la protection d'aiguille distale ou peut être couplé automatiquement à la protection d'aiguille distale lors du déplacement dans la direction proximale ou lors du déplacement dans la direction distale.

16. Dispositif de protection d'aiguille selon l'une quelconque des revendications précédentes, **caractérisé en ce que** la protection d'aiguille (5) distale est mobile de manière à sortir d'une position de départ distale dans la direction proximale jusque dans sa position dégagée et parvient, lors du déplacement dans la direction proximale ou dans la position dégagée ou lors du déplacement dans la direction distale, en prise avec l'organe de déblocage (8 ; 39), laquelle prise entraîne le déplacement de l'organe de déblocage (8 ; 30) dans la position neutre.

17. Dispositif de protection d'aiguille selon la revendication 15 et 16, **caractérisé en ce que** le couplage est réalisé sous la forme d'une prise par entraînement, dans lequel la protection d'aiguille distale entraîne, lors de son déplacement dans la direction distale, l'organe de déblocage jusque dans la position neutre, ou est réalisée sous la forme d'un guidage à coulisse, dans lequel le guidage à coulisse transforme le déplacement dans la direction proximale ou le déplacement dans la direction distale de la protection d'aiguille distale en le déplacement de l'organe de déblocage hors de la position de déblocage dans la position neutre.
